# EUROPEAN PATENT SPECIFICATION

(11) **EP 2 619 321 B1**
(45) Date of publication and mention of the grant of the patent: **01.08.2018**
(21) Application number: 11764045.8
(22) Date of filing: 20.09.2011
(51) Int. Cl.: C12Q 1/68

(54) **BIOMARKERS FOR DIFFERENTIATING MELANOMA FROM BENIGN NEVUS IN THE SKIN**
BIOMARKER ZUR DIFFERENZIERUNG EINES MELANOMS VON EINEM GUTARTIGEN MUTTERMAL AUF DER HAUT
MARQUEURS BIOLOGIQUES PERMETTANT DE DIFFÉRENCIER UN MÉLANOME D'UN NÆVUS BÉNIN SUR LA PEAU

(30) Priority: 20.09.2010 US 384707 P; 01.04.2011 US 201161470682 P
(43) Date of publication of application: 31.07.2013
(73) Proprietor: Advanced Cell Diagnostics, Inc., Newark, CA 94560 (US)
(72) Inventor: MA, Xiao-Jun, Pleasanton, CA 94588 (US); WU, Xingyong, Pleasanton, CA 94566 (US); LUO, Yuling, San Ramon, CA 94582 (US)
(74) Representative: Bezzubova, Olga
(86) International application number: PCT/US2011/052305
(87) International publication number: WO 2012/040168

(56) References cited:
- WO-A2-2006/002433
- HAQQ CHRISTOPHER ET AL: "The gene expression signatures of melanoma progression", PROCEEDINGS OF THE NATIONAL ACADEMY OF SCIENCES OF USA, NATIONAL ACADEMY OF SCIENCE, WASHINGTON, DC; US, vol. 102, no. 17, 26 April 2005 (2005-04-26), pages 6092-6097, XP002562661, ISSN: 0027-8424, DOI: 10.1073/PNAS.0501564102 [retrieved on 2005-04-15]
- CONWAY CAROLINE ET AL: "Gene expression profiling of paraffin-embedded primary melanoma using the DASL assay identifies increased osteopontin expression as predictive of reduced relapse-free survival.", CLINICAL CANCER RESEARCH : AN OFFICIAL JOURNAL OF THE AMERICAN ASSOCIATION FOR CANCER RESEARCH 15 NOV 2009 LNKD- PUBMED:19887478, vol. 15, no. 22, 15 November 2009 (2009-11-15), pages 6939-6946, XP002670883, ISSN: 1078-0432
- HARLIN HELENA ET AL: "Chemokine expression in melanoma metastases associated with CD8+ T-cell recruitment.", CANCER RESEARCH 1 APR 2009 LNKD- PUBMED:19293190, vol. 69, no. 7, 1 April 2009 (2009-04-01), pages 3077-3085, XP002670884, ISSN: 1538-7445
- IKEDA HIDEYUKI ET AL: "Characterization of an antigen that is recognized on a melanoma showing partial HLA loss by CTL expressing an NK inhibitory receptor", IMMUNITY, CELL PRESS, US, vol. 6, no. 2, 1 February 1997 (1997-02-01), pages 199-208, XP002216277, ISSN: 1074-7613, DOI: 10.1016/S1074-7613(00)80426-4
- ALEXANDRESCU DORU T ET AL: "Melanoma-specific marker expression in skin biopsy tissues as a tool to facilitate melanoma diagnosis.", THE JOURNAL OF INVESTIGATIVE DERMATOLOGY JUL 2010 LNKD- PUBMED:20357814, vol. 130, no. 7, July 2010 (2010-07), pages 1887-1892, XP002670885, ISSN: 1523-1747
- KOH STEPHEN S ET AL: "Molecular classification of melanomas and nevi using gene expression microarray signatures and formalin-fixed and paraffin-embedded tissue.", MODERN PATHOLOGY : AN OFFICIAL JOURNAL OF THE UNITED STATES AND CANADIAN ACADEMY OF PATHOLOGY, INC APR 2009 LNKD- PUBMED:19270649, vol. 22, no. 4, April 2009 (2009-04), pages 538-546, XP002670886, ISSN: 1530-0285
- LIU L ET AL: "Detection of circulating cancer cells in lung cancer patients with a panel of marker genes", BIOCHEMICAL AND BIOPHYSICAL RESEARCH COMMUNICATIONS, ACADEMIC PRESS INC. ORLANDO, FL, US, vol. 372, no. 4, 8 August 2008 (2008-08-08), pages 756-760, XP027181572, ISSN: 0006-291X, DOI: 10.1016/J.BBRC.2008.05.101 [retrieved on 2008-06-02]
- PANICI P ET AL: "Predictive value of multiple tumor marker assays in second-look procedures for ovarian cancer", GYNECOLOGIC ONCOLOGY, ACADEMIC PRESS, LONDON, GB, vol. 35, no. 3, 1 December 1989 (1989-12-01), pages 286-289, XP026231880, ISSN: 0090-8258, DOI: 10.1016/0090-8258(89)90064-4 [retrieved on 1989-12-01]

## Description

### FIELD OF INVENTION

Provided herein is a method for detection of melanoma by monitoring the presence and levels of specific biomarkers in samples obtained from patients suspected of melanoma. Also provided herein are methods and kits for diagnosing melanoma and providing prognosis of melanoma development in patients based on the presence and levels of specific biomarkers. Further disclosed herein are methods for determining the stage of melanoma based on the presence of malignant melanoma cells in sentinel lymph nodes of patients.

### BACKGROUND OF THE INVENTION

The incidence of cutaneous melanoma has been increasing rapidly over the past several decades, and it is currently the 5^{th} and 7^{th} most common cancer in men and women, respectively, in the US (Jemal, A., Siegel, R., Xu, J. & Ward, E. (2010) Cancer Statistics, CA Cancer J Clin. 60(5):277-300). Although melanoma represents only 4% of all skin cancers, it accounts for 80% of deaths from all skin cancers. Due to the marked difference in five-year survival probability between metastatic (<15%) and localized disease (98%) (Miller, A.J. & Mihm, M.C. Melanoma. (2006) The New England Journal of Medicine 355, 51-65), early detection and diagnosis is the best approach to improve melanoma outcome.

Melanoma originates from melanocytes, which make the skin pigment melanin. Diagnosis of melanocytic tumors is regarded as one of the most difficult tasks in diagnostic histopathology (Urso, C., Saieva, C., Borgognoni, L., Tinacci, G. & Zini, E. (2008) "Sensitivity and specificity of histological criteria in the diagnosis of conventional cutaneous melanoma" Melanoma research 18, 253-8). This difficulty is reflected in the fact that misdiagnosis of melanoma ranks second on the list of pathology malpractice claims (Troxel, D.B. Medicolegal (2006) "Aspects of error in pathology" Archives of pathology & laboratory medicine 130, 617-9). Both false positive and false negative diagnoses can occur (Veenhuizen, K.C. et al. (1997) "Quality assessment by expert opinion in melanoma pathology: experience of the pathology panel of the Dutch Melanoma Working Party" The Journal of Pathology 182, 266-72). A recent study of melanocytic lesions referred to a specialized clinic showed a discordance rate of 14.3% between the referring pathologists and an expert panel (Shoo, B.A., Sagebiel, R.W. & Kashani-Sabet, M. (2010) "Discordance in the histopathologic diagnosis of melanoma at a melanoma referral center" Journal of the American Academy of Dermatology 62, 751-6). Due to the large number (∼4 million) of biopsies of melanocytic lesions performed annually in the US, this discordant rate can translate into 640,000 ambiguous diagnoses per year (Shoo, B.A., Sagebiel, R.W. & Kashani-Sabet, M. (2010) "Discordance in the histopathologic diagnosis of melanoma at a melanoma referral center" Journal of the American Academy of Dermatology 62, 751-6). The medical and legal pressure to diagnose early melanoma in cases of diagnostic ambiguity may also have contributed to the rapid rise in melanoma incidence (Welch, H.G. & Black, W.C. (2010) "Overdiagnosis in Cancer" Journal of the National Cancer Institute 102, 605-13; Glusac, E.J. (2011) "The melanoma 'epidemic', a dermatopathologist's perspective" Journal of Cutaneous Pathology 38, 264-7). Misdiagnosis can lead to under-treatment or over-treatment for the suspected lesion, adversely impacting patient outcome and quality of life.

The reason for this diagnostic difficulty is that melanocytes also give rise to a variety of benign neoplasms called melanocytic nevi, which can mimic melanoma histologically. Conversely, some malignant melanomas can mimic benign nevi. Furthermore, the recognition of the many histological features used in diagnosis can be subjective and dependent on experience and thus cause poor inter-observer reproducibility. None of the IHC markers being used today in melanoma diagnosis can differentiate between melanoma and benign nevus (Ugurel, S., Utikal, J. & Becker, J.C. (2009) "Tumor biomarkers in melanoma" Cancer Control: Journal of the Moffitt Cancer Center 16, 219-24; Prieto, V.G. & Shea, C.R. (2008) "Use of immunohistochemistry in melanocytic lesions" Journal of Cutaneous Pathology 35 Suppl 2, 1-10). Therefore, there is an urgent need to develop molecular methods to improve diagnostic accuracy.

To address this need, many genomic studies at the DNA (Bastian, B.C., Olshen, A.B., LeBoit, P.E. & Pinkel, D. (2003) "Classifying melanocytic tumors based on DNA copy number changes" The American Journal of Pathology 163, 1765-70; Harvell, J.D., Kohler, S., Zhu, S. & Hernandez-boussard, T. (2004) "High-Resolution Array-Based Comparative Genomic and Melanomas" Diagnostic Molecular Pathology 94305, 22-25) and RNA (Seykora, J.T., Jih, D., Elenitsas, R., Horng, W.-H. & Elder, D.E. (2003) "Gene expression profiling of melanocytic lesions" The American Journal of Dermatopathology 25, 6-11; Talantov, D. et al. (2005) "Novel genes associated with malignant melanoma but not benign melanocytic lesions" Clinical Cancer Research 11, 7234-42; Koh, S.S. et al. (2009) "Molecular classification of melanomas and nevi using gene expression microarray signatures and formalin-fixed and paraffin-embedded tissue" Modern Pathology 22, 538-46; Haqq, C. et al. (2005) "The gene expression signatures of melanoma progression" Proc Natl Acad Sci U S A 102, 6092-6097; Smith, A.P., Hoek, K. & Becker, D. (2005) "Whole-genome expression profiling of the melanoma progression pathway reveals marked molecular differences between nevi/melanoma in situ and advanced-stage melanomas" Cancer Biology & Therapy 4, 1018-29; Scatolini, M. et al. (2010) "Altered molecular pathways in melanocytic lesions" International Journal of Cancer (Journal International Du Cancer) 126, 1869-81) level have been performed to discover novel molecular markers to better distinguish melanoma from benign melanocytic nevi.

By comparing chromosomal DNA copy number changes between melanomas and benign nevi, four genomic loci (6p25, centromere 6, 6q23, and 11q13) were selected to develop a multicolor fluorescence *in situ* hybridization (FISH) assay (Gerami, P. et al. (2009) "Fluorescence in situ hybridization (FISH) as an ancillary diagnostic tool in the diagnosis of melanoma" The American Journal of Surgical Pathology 33, 1146-56). An algorithm based on signal counts (i.e., copy number gains or losses) and percentage of cells with aberrations was shown to have 86.7% sensitivity and 95.4% specificity for melanoma in a validation cohort of unequivocal cases of melanomas and nevi (Gerami, P. et al. (2009) "Fluorescence in situ hybridization (FISH) as an ancillary diagnostic tool in the diagnosis of melanoma" The American Journal of Surgical Pathology 33, 1146-56). However, in classifying histologically ambiguous lesions, when compared to clinical outcome, the sensitivity and specificity of the FISH probes in identifying malignant diseases were 43% and 80%, respectively, in one study (Vergier, B. et al. (2011) "Fluorescence in situ hybridization, a diagnostic aid in ambiguous melanocytic tumors: European study of 113 cases" Mod. Pathol. 24(5):613-23), and 60% and 50% in another (Gaiser, T. et al. (2010) "Classifying ambiguous melanocytic lesions with FISH and correlation with clinical long-term follow up". Modern Pathology 23, 413-9), raising concerns about their clinical utility in classifying difficult cases. This assay is the only commercially available molecular diagnostic test on the market today (MelanoSite™, Neogenomics/Abbot Molecular).

Gene expression microarrays have identified many genes differentially expressed between melanomas and benign nevi (Seykora, J.T., Jih, D., Elenitsas, R., Horng, W.-H. & Elder, D.E. (2003) "Gene expression profiling of melanocytic lesions." The American Journal of Dermatopathology 25, 6-11; Talantov, D. et al. (2005) "Novel genes associated with malignant melanoma but not benign melanocytic lesions" Clinical Cancer Research 11, 7234-42; Koh, S.S. et al. (2009) "Molecular classification of melanomas and nevi using gene expression microarray signatures and formalin-fixed and paraffin-embedded tissue" Modern Pathology 22, 538-46; Haqq, C. et al. (2005) "The gene expression signatures of melanoma progression" Proc. Natl. Acad. Sci. U S A 102, 6092-6097; Smith, A.P., Hoek, K. & Becker, D. (2005) "Whole-genome expression profiling of the melanoma progression pathway reveals marked molecular differences between nevi/melanoma in situ and advanced-stage melanomas" Cancer Biology & Therapy 4, 1018-29; Scatolini, M. et al. (2010) "Altered molecular pathways in melanocytic lesions. International journal of cancer" Journal International Du Cancer 126, 1869-81). Different approaches were taken to translate these discoveries into clinical diagnostic tools. Talantov et al. selected three genes (GDF15, SILV and L1CAM) from their microarray analysis and analyzed their expression in an independent cohort by reverse transcription real-time PCR (RT-PCR); but only one of these genes, SILV, demonstrated modest performance (AUC=0.74; sensitivity ∼40% at 90% specificity) in distinguishing melanoma from atypical nevi (Alexandrescu, D.T. et al. (2010) "Melanoma-specific marker expression in skin biopsy tissues as a tool to facilitate melanoma diagnosis" J. Invest. Dermatol. 130, 1887-1892). Koh et al. developed a microarray-based 37-gene classifier, which demonstrated 89% concordance with histopathology in differentiating melanoma from benign nevi (Koh, S.S. et al. (2009) "Molecular classification of melanomas and nevi using gene expression microarray signatures and formalin-fixed and paraffin-embedded tissue" Modern Pathology 22, 538-46). Kashani-Sabet and coworkers used the results of their microarray studies to develop a 5-gene (ARPC2, FN1, RGS1, SPP1, and WNT2) immunohistochemistry (IHC) panel, which demonstrated 91% sensitivity and 95% specificity for melanoma (Kashani-Sabet, M. et al. (2009) "A multi-marker assay to distinguish malignant melanomas from benign nevi" Proc. Natl. Acad. Sci. U S A 106, 6268-6272); however, it is important to point out that these values were based on the entire training dataset and thus likely to be significantly overestimated.

These different approaches of translating differential gene expression into clinical diagnostics all have significant limitations. Both RT-PCR and microarray methods require RNA extraction, and the resulting gene expression measurements represent an "average" of heterogeneous groups of cells. Careful microdissection can help, but it is too cumbersome for routine practice. IHC is an attractive approach to provide cellular resolution, but translating differential gene expression at the RNA level to IHC can be difficult, because the best candidate markers may not have suitable antibodies for IHC. This means either a major undertaking to develop new antibodies or use of a suboptimal gene panel based on antibody availability.

Because of the great cellular heterogeneity in a typical melanocytic lesion, RNA *in situ* hybridization (ISH) is the preferred method for gene expression analysis, since it can be used to determine gene expression at single cell resolution Suzuki, I. & Motokawa, T. (2004) "In situ hybridization: an informative technique for pigment cell researchers" Pigment Cell Research 17, 10-4). However, existing RNA ISH methods have poor sensitivity and specificity and are technically demanding, making them impractical for most of the genes identified in microarray analysis.

A novel RNA ISH technology platform called RNAscope® was recently developed. RNAscope® has single-molecule sensitivity, uses a 6-hour IHC-like procedure, and is multiplex-capable, and compatible with formalin-fixed paraffin-embedded (FFPE) tissue. This makes it possible for the first time to rapidly develop RNA ISH assays for any candidate genes identified from microarrays.

The importance of tumor microenvironment in tumor invasion and metastasis has been firmly established (Hanahan, D. & Weinberg, R.A. (2011) "Hallmarks of Cancer: The Next Generation" Cell 144, 646-674). However, to date cancer diagnostics has either focused exclusively on tumor cell-intrinsic markers (e.g., see Gerami, P. et al. (2009) "Fluorescence in situ hybridization (FISH) as an ancillary diagnostic tool in the diagnosis of melanoma" The American Journal of Surgical Pathology 33, 1146-56) or lack the ability to interrogate tumor and stroma separately in a practical manner (e.g., see Paik, S. et al. (2004) "A multigene assay to predict recurrence of tamoxifen-treated, node-negative breast cancer" The New England Journal of Medicine 351, 2817-26; van't Veer, L.J. et al. (2002) "Gene expression profiling predicts clinical outcome of breast cancer" Nature 415, 530-6; Finak, G. et al. (2008) "Stromal gene expression predicts clinical outcome in breast cancer" Nature Medicine 14, 518-27). For example, a stroma-derived gene expression signature for breast cancer prognosis has been identified before (Finak, G. et al. (2008) "Stromal gene expression predicts clinical outcome in breast cancer" Nature Medicine 14, 518-27). Its implementation as a microarray- or RT-PCR-based test for clinical practice will be challenging since it will require careful microdissection of the stroma from the tumor and it cannot differentiate the many stromal cell types. The role of tumor-host interactions in melanoma progression is well recognized (Hsu, M.-Y., Meier, F. & Herlyn, M. (2002) "Melanoma development and progression: a conspiracy between tumor and host" Differentiation 70, 522-36). Alterations in expression of growth factors, receptors and ECM proteins take place early and work in concert with the accumulative genetic changes in melanocytes to promote melanoma progression. Therefore, the tumor-stroma interface offers abundance of targets for diagnostics, which can be exploited by RNAscope®.

The first RNA ISH-based multi-gene assay for the diagnosis of malignant melanoma and benign nevus was developed in the present application. This assay determines the expression of PHACTR1 gene either alone or in combination with one or more of SPP1, PRAME, GDF15, and/or CXCL10 genes at cellular resolution. In contrast to earlier approaches noted above, RNAscope® enables a novel biomarker strategy which detects gene expression changes in both the tumor cells and tumor-associated stroma for increased clinical sensitivity and specificity for early stage melanoma. The assays developed in the present invention allow pathologists to seamlessly integrate novel molecular markers with conventional histopathology, significantly advancing today's clinical practice in melanoma diagnosis. Ultimately, the tests result in more accurate diagnosis of early melanoma while minimizing false positives to spare patients with benign lesions unnecessary treatment and follow-ups.

### SUMMARY OF THE INVENTION

Therefore, an object of the present invention is to provide new markers for diagnosing the presence of melanoma or for providing a prognosis of the likelihood of recurrence of melanoma, in particular in humans, and for determining the staging of melanoma using such markers.

In a first aspect, the invention provides a method for diagnosing melanoma in a human subject suspected of melanoma, comprising determining the level of expression of integrin-binding phosphoprotein (SPP1) gene, or fragments thereof and preferentially expressed antigen in melanoma (PRAME) gene, or fragments thereof in a tissue sample from said human subject by *in situ* hybridization, wherein said tissue sample comprises a plurality of melanocytes, thereby diagnosing the presence of melanoma based on the expression levels in said tissue sample.

In a second related aspect, the invention provides a method for providing a prognosis to a human subject who is at risk of developing melanoma recurrence, comprising determining the level of expression of integrin-binding phosphoprotein (SPP1) gene, or fragments thereof and preferentially expressed antigen in melanoma (PRAME) gene, or fragments thereof in a tissue sample from said human subject by *in situ* hybridization, wherein said tissue sample comprises a plurality of melanocytes, thereby providing prognosis based on the expression levels in said tissue sample.

In one embodiment, said determining the level of expression further comprises determining the level of expression of phosphatase and actin regulator 1 (PHACTR1) gene, or fragments thereof; growth differentiation factor 15 (GDF15) gene, or fragments thereof; and chemokine C-X-C motif ligand 10 (CXCL10) gene, or fragments thereof.

In another embodiment, said tissue sample is skin.

In another embodiment, said diagnosing, or said providing prognosis is based on a pattern of expression levels of said genes. In a further embodiment, said expression levels of said genes are determined individually; or are pooled and determined altogether.

In another embodiment, said determining the level of expression comprises detecting the expression level of mRNA expressed from said genes; or detecting the expression level of polypeptides encoded by said genes.

In another embodiment, the upregulation of the mRNA expression of said genes, or the upregulation of the polypeptide expression of said genes is indicative of melanoma, or of recurrence of melanoma, or of risk of recurrence of melanoma.

In another aspect, the invention provides a kit for the diagnosis or prognosis of melanoma, comprising target probes for the *in situ* hybridization detection of the level of expression of the integrin-binding phosphoprotein (SPP1) target gene, or fragments thereof, and preferentially expressed antigen in melanoma (PRAME) gene, or fragments thereof, wherein the target probes are polynucleotides that are capable of hybridizing to an RNA expressed from the target genes and capturing label probes to said RNA.

In one embodiment, said kit further comprises target probes and label probes for the in situ hybridization detection of the expression level of the phosphatase and actin regulator 1 (PHACTR1) gene, or fragments thereof, growth differentiation factor 15 (GDF15) gene, or fragments thereof, and chemokine C-X-C motif ligand 10 (CXCL10) gene, or fragments thereof.

In another embodiment, the kit further comprises preamplifiers or amplifiers, wherein an amplifier is a polynucleotide that is capable of hybridizing to multiple label probes and at least one target probe or a nucleic acid bound to a target probe, and wherein a preamplifier is a polynucleotide that serves as an intermediate between one or more target probes and amplifiers.

In a further aspect, the invention provides a kit for the diagnosis or prognosis of melanoma, comprising ligands or antibodies for detection of the level of target polypeptides, wherein said target polypeptides comprise a target polypeptide encoded by integrin-binding phosphoprotein (SPP1) gene, or fragments thereof; and a target polypeptide encoded by preferentially expressed antigen in melanoma (PRAME) gene, or fragments thereof.

In one embodiment, said target polypeptides further comprise a target polypeptide encoded by phosphatase and actin regulator 1 (PHACTR1) gene, or fragments thereof; a target polypeptide encoded by growth differentiation factor 15 (GDF15) gene, or fragments thereof; and a target polypeptide encoded by chemokine C-X-C motif ligand 10 (CXCL10) gene, or fragments thereof.

In yet another aspect, the invention provides a kit for the diagnosis or prognosis of melanoma, comprising oligonucleotides for the *in situ* hybridization detection of the expression level of the integrin-binding phosphoprotein (SPP1) target gene, or fragments thereof and preferentially expressed antigen in melanoma (PRAME) gene, or fragments thereof, wherein the oligonucleotides are capable of hybridizing to an RNA expressed from said target genes.

In one embodiment, said kit further comprises oligonucleotides for the *in situ* hybridization detection of the expression level of the phosphatase and actin regulator 1 (PHACTR1) gene, or fragments thereof, growth differentiation factor 15 (GDF15) gene, or fragments thereof, and chemokine C-X-C motif ligand 10 (CXCL10) gene, or fragments thereof.

Disclosed herein is a method for diagnosing melanoma in a subject, comprising steps of providing a tissue sample from a human subject, wherein the tissue sample comprises a plurality of melanocytes, and determining the level of expression of phosphatase and actin regulator 1 (PHACTR1) marker, or fragments thereof, thereby diagnosing the presence of melanoma based on the expression levels in the provided tissue. In this case, PHACTR1 may be used either alone or in combination with SPP1, PRAME, GDF15, and/or CXCL10 as markers. PHACTR1 may be used in combination with SPP1 as markers.

By means of comparative analyses between malignant tissue and non-malignant tissue, genes and proteins can be identified that differ significantly in the types of tissue in their frequency and/or concentration. The quantitative expression of a particular gene or protein in comparison with controls, particularly of the genes and proteins claimed in this invention, thus constitutes an important indicator for the presence of tumor tissues, particularly melanoma, which makes it possible to use these genes and/or proteins as diagnostic markers.

In experiments the inventions is based upon, the inventor discovered that PHACTR1, SPP1, PRAME, GDF15, and/or CXCL10 genes/proteins, in particular PHACTR1 and SPP1 genes/proteins, are well-suited for use as markers for the diagnosis/prognosis of whether or not the tissue sample tested has melanoma.

Disclosed herein is a method for diagnosing the presence of melanoma, in which upregulation of PHACTR1, either alone or in combination with upregulation of SPP1, PRAME, GDF15, and/or CXCL10, is indicative of melanoma. PHACTR1 may be used in combination with SPP1 as makers.

According to one aspect of the invention, determination of the levels of expression of the genes comprises detecting either of the expression level of mRNA expressed form said genes or of the expression level of polypeptides encoded by said genes. In a preferred embodiment, the detection of level of expression of mRNA is performed *in situ.*

In particular, upregulation of the PHACTR1 gene expression is determined in combination with the upregulation of SPP1, PRAME, GDF15, and/or CXCL10, in comparison to gene/protein expression of the above genes in normal (or non-malignant) tissue.

In one aspect of the invention, diagnosis of the presence of melanoma is based on the pattern of expression levels of the markers claimed in the present invention. In one embodiment, the level of expression of each marker in the set of markers are determined individually. Based on the pattern of different expression levels of these markers, a diagnosis of the presence of melanoma or a prognosis of the likelihood of the recurrence of melanoma is made. In another embodiment, each marker may be given a weight, based on its power of influence in the analysis. The weighted expression level of the markers are then summed up to produce a score. The score is then used for providing diagnosis or prognosis of melanoma based on a threshold.

In yet another embodiment, the level of expression of each marker in the set of markers are determined indistinctively, *e.g.*, by using an identical label for different markers. The expression level of the markers are pooled and determined altogether. Diagnosis or prognosis of melanoma is then made based on the accumulative measurement.

In addition to diagnosing melanoma and providing prognosis of melanoma by quantitatively measuring the level of expression of the markers claimed in the present invention, a method of qualitatively diagnosing melanoma and providing prognosis of melanoma based on the present or the absence of the markers is also provided in the present invention. In one embodiment, the presence of PHACTR1 marker in combination with the presence of SPP1, PRAME, GDF15, and CXCL10 markers, is indicative of the presence or the recurrence of melanoma.

The protein sequence of the marker PHACTR1 as well as the gene sequence coding for SPP1, PRAME, GDF15, and CXCL10 are listed in publicly available databases. For example, the mRNA sequences of these genes are included in the database ("GenBank") of the National Center for Biotechnology Information (NCBI: http:/(www).ncbi.nlm.nih.gov/) under database No. NM_030948.1 for PHACTR1, NM 000582.2 for SPP1, NM 006115.3 for PRAME, NM_004864.2 for GDF15, NM 001565 for CXCL10, NM 005511.1 for MLANA, NM_014624.3 for S 100A6, and NM 006272.2 for S100B. The gene and protein sequences corresponding to the markers can also be identified via these mRNA numbers, so the present invention expressly include the respective sequences, or fragments thereof, published in the databases and used in the claimed methods.

The mRNA sequences of the diagnostic markers disclosed in the present invention are identified as SEQ ID NO. 1 for PHACTR1, SEQ ID NO. 3 for GDF15, SEQ ID NO. 5 for MLANA, SEQ ID NO. 7 for CXCL10, SEQ ID NO. 9 for PRAME, SEQ ID NO. 11 for S100A6, SEQ ID NO. 13 for S100B, and SEQ ID NO. 15 for SPP1. The protein sequence of the same markers are identified as SEQ ID NO. 2 for PHACTR1, SEQ ID NO. 4 for GDF15, SEQ ID NO. 6 for MLANA, SEQ ID NO. 8 for CXCL10, SEQ ID NO. 10 for PRAME, SEQ ID NO. 12 for S100A6, SEQ ID NO. 14 for S100B, and SEQ ID NO. 16 for SPP1.

The markers can be qualitatively or quantitatively determined at the mRNA level via any gene expression analysis technologies which measure mRNA in solution. Examples of such gene expression analysis technologies include, but not limited to, RNAscope®, RT-PCR, Nanostring, QuantiGene® 2.0, qNPA™, microarray, and sequencing.

In addition to diagnosing the presence of melanoma, the methods of the invention can be used for differentiating melanoma from benign nevus. According to one aspect, the presence of PHACTR1 markers and SPP1, PRAME, GDF15, and CXCL10 markers is indicative of the presence of melanoma but the absence of benign nevus in the tested human subject. In another aspect, the presence of PRAME PHACTR1 and SPP1 markers is indicative of the presence of melanoma. In yet another aspect, the absence of PHACTR1 and SPP1 markers but the presence of MLANA marker is indicative of benign nevus in the tested human subject.

The present disclosure also concerns a method of determining the stage of melanoma in a human subject. The method comprises the steps of obtaining a tissue sample from a human subject, wherein the tissue sample comprises sentinel lymph node; determining the level of expression of PHACTR1 marker, either alone or in combination one or more of SPP1, PRAME, GDF15, and CXCL10 markers, and determining the stage of melanoma as characterized by the presence of malignant melanoma cells in sentinel lymph node based on the expression levels in the tissue sample. In another instance the method uses a combination of PHACTR1 and SPP1 markers.

As mentioned above, in the method of determining the stage of melanoma, determination may be carried out using the level of expression of mRNA expressed from the genes or the level of expression polypeptides encoded by the genes. In addition, a pattern of the level of expression of the markers claimed in the present invention can be used for determining the stage of melanoma. When a pattern of the expression levels is used, the levels of expression of the markers are determined individually.

In the method of determining the stage of melanoma, upregulation of expression levels of markers PHACTR1, SPP1, PRAME, GDF15, and/or CXCL10 predicts recurrence of melanoma. The markers can be qualitatively or quantitatively determined at the mRNA level via any gene expression analysis technologies which measure mRNA in solution, including, but not limited to RNAscope®, RT-PCR, Nanostring, QuantiGene® 2.0, qNPA™, and microarray.

The invention also concerns diagnostic kits comprising at least one substance for detecting the expression of PHACTR1 marker in combination with SPP1, PRAME, GDF15, and CXCL10 markers for the diagnosis and prognosis of melanoma. In one embodiment, the diagnostic kit comprises substances for detecting the expression of PRAME and SPP1 markers.

The diagnostic kits can be advantageously used to determine the quantity of the markers according to the invention in biological samples, namely by comparing the gene/protein expression of the sample of interest that contains the marker or markers with controls or standards, specifically with respect to increased expression of the marker(s). The diagnostic kit contains e.g. one or more antibodies or oligonucleotides that react with the marker/marker combinations according to the invention at the protein or gene level.

### BRIEF DESCRIPTION OF THE DRAWINGS

**Fig.1** illustrates the principle of the RNAscope® assay. Step 1: Cells or tissues are fixed and permeablized to allow for target probe access. Step 2: Target RNA-specific oligonucleotide probes are hybridized to multiple mRNAs. Step 3: Multiple signal amplification molecules that each recognize a specific target probe are hybridized. Each unique label probe is conjugated to a different fluorophore or enzyme. Step 4: Signals are detected using a standard bright-field or epifluorescent microscope.
**Fig. 2** illustrates single copy mRNA detection by RNAscope®. (a) HER2 DNA FISH of HeLa and SK-BR-3 nuclei with RNAscope® target probes and signal amplifiers. Nuclei were co-stained with IL-8 probes as an internal control for diploid gene copy number. Nuclei counterstained with DAPI. (b) Target probes to Her-2 mRNA are hybridized with HeLa cells (left) or SK-BR-3 cells (right). Cells were co-stained with 18S rRNA target probes as an internal control for assay success.
**Fig. 3** illustrates chromogenic detection of mRNA in FFPE by RNAscope®. Breast, lung and prostate FFPE tissue sections hybridized with probes either for UBC (top row, - Figs. 3A, 3C, 3E) or a bacterial gene DapB (bottom row, - Figs. 3B, 3D, 3F). UBC staining was detected in all three tissues; no staining was seen with DapB. Nuclei were counterstained with hematoxylin.
**Fig. 4** illustrates up-regulation of PHACTR1 in melanoma in two microarray datasets. P values were from Kruskal-Wallis test.

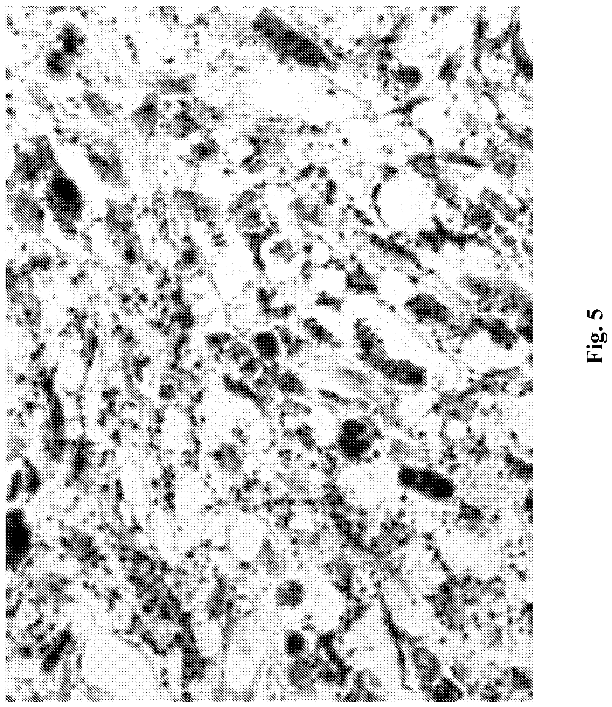

**Fig. 5** illustrates deteion of mRNAs in melanoma. PHACTR1 mRNA detected as individual red dots. Hematoxylin staining of nuclei (blue) and natural melanin background (brown) are also shown.
**Fig. 6** illustrates an example of staining patterns in a nevus (left, - Figs. 6A, 6B, 6C) and a melanoma (right, - Figs. 6D, 6E, 6F). Positive mRNA staining were detected as red dots. Blue, hematoxylin staining of nuclei.
**Fig. 7** illustrates an example of SPP1 mRNA staining in stromal cells. SPP1. Positive mRNA staining was detected as red dots in scattered stromal cells within the tumor. Blue, hematoxylin staining of nuclei.
**Fig. 8** illustrates RNAscope® staining of a desmoplastic melanoma. The probes for S100A6 and S100B were pooled to detect both together. RNA staining are shown in red. Nuclei are shown in blue.
**Fig. 9** illustrates examples of positive staining patterns of GDF15 (Fig. 9A), PRAME (Fig. 9B) and CXCL10 (Fig. 9C) in melanomas. RNA staining is shown in red. Nuclei are shown in blue.

### DETAILED DESCRIPTION

Provided herein are methods based, in part, on the discovery that the presence and level of certain molecules or mRNA in patient samples can be utilized as biomarkers to indicate the presence of melanoma. In particular, these biomarkers can be used to differentiate melanoma from benign nevus in the skin.

### 1. Definitions

Unless defined otherwise, all technical and scientific terms used herein have the same meaning as commonly understood by one of ordinary skill in the art to which the invention pertains. The following definitions supplement those in the art and are directed to the current application and are not to be imputed to any related or unrelated case, *e.g.,* to any commonly owned patent or application. Although any methods and materials similar or equivalent to those described herein can be used in the practice for testing of the present invention, the preferred materials and methods are described herein. Accordingly, the terminology used herein is for the purpose of describing particular embodiments only, and is not intended to be limiting.

The term "polynucleotide" (and the equivalent term "nucleic acid") encompasses any physical string of monomer units that can be corresponded to a string of nucleotides, including a polymer of nucleotides (*e.g*., a typical DNA or RNA polymer), peptide nucleic acids (PNAs), modified oligonucleotides (*e.g.*, oligonucleotides comprising nucleotides that are not typical to biological RNA or DNA, such as 2'-O-methylated oligonucleotides), and the like. The nucleotides of the polynucleotide can be deoxyribonucleotides, ribonucleotides or nucleotide analogs, can be natural or non-natural, and can be unsubstituted, unmodified, substituted or modified. The nucleotides can be linked by phosphodiester bonds, or by phosphorothioate linkages, methylphosphonate linkages, boranophosphate linkages, or the like. The polynucleotide can additionally comprise non-nucleotide elements such as labels, quenchers, blocking groups, or the like. The polynucleotide can be, *e.g.*, single-stranded or double-stranded.

A "nucleic acid target" or "target nucleic acid" refers to a nucleic acid, or optionally a region thereof, that is to be detected.

A "polynucleotide sequence" or "nucleotide sequence" is a polymer of nucleotides (an oligonucleotide, a DNA, a nucleic acid, etc.) or a character string representing a nucleotide polymer, depending on context. From any specified polynucleotide sequence, either the given nucleic acid or the complementary polynucleotide sequence (*e.g.*, the complementary nucleic acid) can be determined.

The term "gene" is used broadly to refer to any nucleic acid associated with a biological function. Genes typically include coding sequences and/or the regulatory sequences required for expression of such coding sequences. The term gene can apply to a specific genomic sequence, as well as to a cDNA or an mRNA encoded by that genomic sequence.

The term "biomarker" and "marker" as used interchangeably herein, refer to both the protein/gene product in question and the gene coding for this product. The term means a protein, gene product, and/or gene of PHACTR1, SPP1, PRAME, GDF15, and CXCL10.

As used herein the terms "polypeptide" and "protein" as used interchangeably herein, refer to a polymer of amino acids of three or more amino acids in a serial array, linked through peptide bonds. The term "polypeptide" includes proteins, protein fragments, protein analogues, oligopeptides and the like. The term polypeptide as used herein can also refer to a peptide. The amino acids making up the polypeptide may be naturally derived, or may be synthetic. The polypeptide can be purified from a biological sample.

The term "antibody" is used herein in the broadest sense and covers fully assembled antibodies, antibody fragments which retain the ability to specifically bind to the antigen (*e.g.*, Fab, F(ab')2, Fv, and other fragments), single chain antibodies, diabodies, antibody chimeras, hybrid antibodies, bispecific antibodies, humanized antibodies, and the like. The term "antibody" covers both polyclonal and monoclonal antibodies.

The term "biological sample" or "tissue sample" as used herein refers to a sample obtained from a biological subject, including sample of biological tissue or fluid origin, obtained, reached, or collected *in vivo* or *in situ.* A biological sample also includes samples from a region of a biological subject containing precancerous or cancer cells or tissues. Such samples can be, but are not limited to, organs, tissues, fractions and cells isolated from a mammal. Exemplary biological samples include but are not limited to cell lysate, a cell culture, a cell line, a tissue, an organ, an organelle, a biological fluid, and the like. Preferred biological samples include but are not limited to a skin sample, tissue biopsies, and the like.

The term "upregulation" or "increased expression" refers to any gene or protein expression that is increased or enhanced compared to normal expression of the gene in question in healthy samples. Comparison of the differing levels of expression may be carried out using controls or standards. "Upregulated" or "increased" gene expression means that the gene in question is transcribed--in and if applicable, translated into the corresponding protein--to a greater extent than is normal for this gene.

A "label" is a moiety that facilitates detection of a molecule. Common labels in the context of the present invention include fluorescent, luminescent, light-scattering, and/or colorimetric labels. Suitable labels include enzymes and fluorescent moieties, as well as radionuclides, substrates, cofactors, inhibitors, chemiluminescent moieties, magnetic particles, and the like. Patents teaching the use of such labels include U.S. Pat. Nos. 3,817,837; 3,850,752; 3,939,350; 3,996,345; 4,277,437; 4,275,149; and 4,366,241. Many labels are commercially available and can be used in the context of the invention.

A "target probe" is a polynucleotide that is capable of hybridizing to a target nucleic acid and capturing a label probe to that target nucleic acid. The target probe can hybridize directly to the label probe, or it can hybridize to one or more nucleic acids that in turn hybridize to the label probe; for example, the target probe can hybridize to an amplifier or a preamplifier. The target probe thus includes a first polynucleotide sequence that is complementary to a polynucleotide sequence of the target nucleic acid and a second polynucleotide sequence that is complementary to a polynucleotide sequence of the label probe, amplifier, preamplifier, or the like. The target probe is preferably single-stranded.

An "amplifier" is a molecule, typically a polynucleotide, that is capable of hybridizing to multiple label probes. Typically, the amplifier hybridizes to multiple identical label probes. The amplifier also hybridizes to at least one target probe or nucleic acid bound to a target probe. For example, the amplifier can hybridize to at least one target probe and to a plurality of label probes, or to a preamplifier and a plurality of label probes. The amplifier can be, *e.g.,* a linear, forked, comb-like, or branched nucleic acid. As noted for all polynucleotides, the amplifier can include modified nucleotides and/or nonstandard internucleotide linkages as well as standard deoxyribonucleotides, ribonucleotides, and/or phosphodiester bonds. Suitable amplifiers are described, for example, in USPN 5,635,352, USPN 5,124,246, USPN 5,710,264, and USPN 5,849,481.

A "preamplifier" is a molecule, typically a polynucleotide, that serves as an intermediate between one or more target probes and amplifiers. Typically, the preamplifier hybridizes simultaneously to one or more target probes and to a plurality of amplifiers. Exemplary preamplifiers are described, for example, in USPN 5,635,352 and USPN 5,681,697.

### 2. Biomarkers

Provided herein are methods relating to the use of mRNA or protein, and other cell marker molecules as biomarkers to detect the presence of melanoma. The presence of biomarkers or their levels can be used to determine whether a given sample has melanoma.

A biological marker or "biomarker" is a substance whose detection indicates a particular biological state, such as, for example, the presence of melanoma. As disclosed herein, biomarkers can either be determined individually, or several biomarkers can be measured simultaneously.

In the research that led to the present invention, a plurality of genes have been identified as associating with melanoma. It has furthermore been demonstrated that specific combinations of these genes are associated with melanoma. The plurality of genes associating with melanoma include, but not limited to: PHACTR1 gene (phosphatase and actin regulator 1), SSP1 gene (secreted integrin-binding phosphoprotein), PRAME gene (preferentially expressed antigen in melanoma), GDF15 gene (growth differentiation factor 15), and CXCL10 gene (chemokine C-X-C motif ligand 10). In addition, a plurality of genes have been identified for using as control genes, including: MLANA (melanoma antigen recognized by T-cells), S100A6, S100B, and DapB.

### 2.1 Selection of PHACTR1, SPP1, and MLANA as Biomarkers for Detecting Melanoma

It was found by the inventors of the present invention that PHACTR1 and SPP1 are the strongest markers for melanoma. PHACTR1 and SPP1 genes were detected at the early phase of melanoma.

In the research that led to the present invention, the gene expression profile of a large number of genes from skin samples ranging from normal skin to benign nevus to melanoma was analyzed. In the samples with abnormal skin condition, the lesions on the skin represented different stages of melanoma progression according to a current model. The model posits that melanoma initiates from dysplastic nevi, which then progresses to melanoma *in situ* or minimally invasive melanoma (RGP) to invasive melanoma (VGP) to metastatic melanoma (MTS) in a stepwise fashion (Hsu, M.-Y., Meier, F. & Herlyn, M. (2002) "Melanoma development and progression: a conspiracy between tumor and host." Differentiation 70(9-10):522-36). The microarray data of genome-wide gene expression analysis were retrieved from the Gene Expression Omnibus (GEO) database (http://(www.)ncbi.nlm.nih.gov/geo/). The GEO database stores gene expression microarray datasets contributed by various research groups, often after bioinformatics and statistical analysis have been conducted on these datasets by the original authors. In order to identify consistent, cross-validated candidate markers, datasets from two different microarray platforms, GSE3189 (Affymetrix® GeneChip® HG-U133A, 22,000 genes) and GSE12391 (Agilent® Human Whole Genome Oligo Microarray, 41,000 genes), were chosen by the inventors for candidate identification.

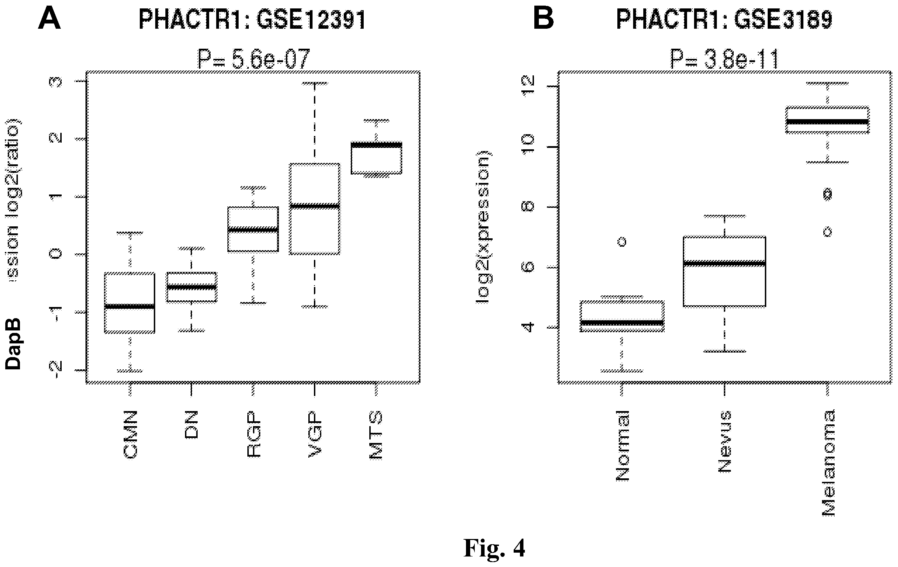

In the present invention, it has been surprisingly found that increased expression level of PHACTR1 gene indicates skins samples having melanoma. Before the present invention, PHACTR1 gene has never been selected as a biomarker for detection or prognosis of melanoma in any studies. In the present invention, PHACTR1 gene was identified as biomarker for melanoma based on the result of novel statistical algorithm and classification method. Limma linear models (Smyth, G.K. Limma (2005) "Linear models for microarray data. Bioinformatics and Computational Biology Solutions using R and Bioconductor" R. Gentleman, V. Carey, S. Dudoit, R. Irizarry, W. Huber (eds.), Springer, New York, pages 397-420) for microarrays was used to find genes differentially expressed between normal skin/benign nevi and melanomas. Limma linear models were developed by Smyth G. K. Limma. This algorithm uses empirical Bayes shrinkage of gene-wise residuals to ensure stable linear models when the number of samples is small, as is the case in the present invention. Using the Limma linear models, PHACTR1 was identified as the most strongly up-regulated gene in microarray GSE12391 and the second most strongly up-regulated gene in melanoma in microarray GSE3189 (see **Table 1**). The p values were adjusted after controlling for multiple hypothesis testing. PHACTR1 demonstrated up-regulation in the early stage of minimally invasive melanoma, whereas its expression in dysplastic nevi was similar to that in common nevi (**Fig. 4**), suggesting its utility to differentiate between dysplastic nevi and melanomas.

In one instance, the present disclosure concerns the use of expression level of PHACTR1 and SPP1 markers to predict whether a skin sample has melanoma. It was surprisingly found that increased expression level of SPP1 marker in combination with increased expression level of PHACTR1 marker indicate the presence of melanoma in the skin samples tested. Thus, the SPP1 gene is a second marker for diagnosing melanoma. Together, the PHACTR1 gene and SPP1 gene, are the strongest markers for melanoma and were detected at the early phase of melanoma. It was found in the present invention that, as shown in **Table 2,** among the 78 melanoma samples tested, 83% and 91% of the melanomas were positive for PHACTR1 and SPP1, respectively, whereas none of the 19 nevi tested had detectable signals for either marker. In combination, 95% of the melanomas were positive for at least one marker: 84% were positive for both, 12% and 4% were positive for either SPP1 or PHACTR1 alone, respectively.

Both SPP1 and PHACTR1 genes involve with tumor invasion and metastasis. SPP1 (osteopontin or OPN) is a secreted integrin-binding phosphoprotein and a component of the extracellular matrix (ECM). Its overexpression has been associated with tumor progression and metastasis in multiple cancer types including melanoma (Wai, P.Y. & Kuo, P.C. (2008) "Osteopontin: regulation in tumor metastasis" Cancer Metastasis Reviews 27, 103-18; Shevde, L.A., Das, S., Clark, D.W. & Samant, R.S. (2010) "Osteopontin: an effector and an effect of tumor metastasis" Current Molecular Medicine 10:71-81; El-Tanani, M.K. et al. (2006) "The regulation and role of osteopontin in malignant transformation and cancer" Cytokine & Growth Factor Reviews 17:463-74; Zhou, Y. et al. (2005) "Osteopontin expression correlates with melanoma invasion" The Journal of Investigative Dermatology 124:1044-52).

The myriad functions of SPP1 include cell-ECM adhesion as a ligand for alpha (-v) -beta containing integrins and CD44 and as an activator of matrix metalloproteases (MMP2 in particular) for ECM remodeling, both of which are important in tumor invasion. The PHACTR1 protein is a member of the PHACTR/scapinin family, which binds to both protein phosphatase 1 (PP1) and actin. Expression of PHACTR3 enhances cell spreading and motility through its PP1-binding and actin-binding activities (Sagara, J., Arata, T. & Taniguchi, S. (2009) "Scapinin, the protein phosphatase 1 binding protein, enhances cell spreading and motility by interacting with the actin cytoskeleton" PLoS One 4, e4247).

Without being limited by theory, it is likely that PHACTR1 may function to regulate tumor invasion and migration. Comparing to benign nevi, a defining hallmark of malignant melanoma is the ability to invade into the dermis and metastasize to distant sites. Therefore, the mechanistic involvement in tumor invasion and metastasis by SPP1 and PHACTR1 may explain their utility as markers for discriminating melanoma from benign nevi. There has not yet been any connection described between the expression level of PHACTR1 and the diagnosis/prognosis of melanoma. There was also no connection described between the expression level of a set of markers consisting of PHACTR1 and SPP1 and the diagnosis/prognosis of melanoma. There was no teaching of a *in situ* hybridization assay using a combination of PHACTR1 and SPP1 markers for the diagnosis/prognosis of melanoma.

Further disclosed herein is the use of MLANA gene as a positive control for the detection of melanoma. MLANA gene expresses in both normal melanocytes and malignant melanocytes such as melanoma cells but not in other type of cells in the skin. Thus MLANA can be used as a biomarker for cell type identification. The presence of MLANA is indicative of the presence of either normal melanocytes or melanoma cells or both. The absence of MLANA is indicative the absence of either normal melanocytes or melanoma cells. If a sample tissue is detected to have the presence of MLANA marker but the absence of PHACTR1 and SPP1 markers, the sample tissue is considered to have benign nevus.

MLANA can also be used in the present invention as a positive control for assay success. Very often detection of markers is performed on Formalin-Fixed, Paraffin-Embedded (FFPE) tissue microarrays (TMAs). These TMAs were often constructed from archival tissues from various times and sources, and thus could vary in the quality of RNA preservation. If MLANA can be detected in a FFPE tissue sample, it indicates that the sample must have acceptable quality. Thus, MLANA also serves as a positive control for RNA integrity.

### 2.2 Detection of Melanoma Using Biomarkers of PHACTR1, SPP1 and/or MLANA

Disclosed herein are methods for detection of melanoma cells in a sample based on the expression level of PHACTR1, SPP1 and MLANA markers. An upregulated level of expression of PHACTR1 and SPP1 markers is indicative of the presence of melanoma. Detection of changes in the level of expression of markers often depends on the sensitivity of the assay used. In an assay with sufficiently high sensitivity, the presence of PHACTR1 and SPP1 markers is indicative of the presence of melanoma. Samples tested with unchanged level of or in absence of PHACTR1 and SPP1 markers but with upregulated level or with the presence of MLANA marker are considered to have benign nevus. Samples tested upregulated or with the presence of either PHACTR1 marker or SPP1 marker or both markers, and also upregulated or with the presence of MLANA marker are considered to have melanoma. MLANA gene serves as positive control for assay success and a marker for melanocytes. In addition, bacterial gene DapB is used as negative control. For example, the expression of PHACTR1 and SPP1 may be determined as positive or negative using the DapB staining as a threshold.

Discloses herein is a method for diagnosing melanoma in a human subject suspected of melanoma, comprising the following steps:
(a) obtaining a tissue sample from said human subject, wherein said tissue sample comprises a plurality of melanocytes;
(b) determining the level of expression of PHACTR1 and SPP1 genes, or fragments thereof; and
thereby diagnosing the presence of melanoma based on the expression levels in said tissue sample.

Disclosed herein is a method for detecting melanoma in a human subject, wherein the presence of either PHACTR1 or SPP1 gene is indicative of melanoma in said human subject.

The detecting step of the above method further comprises detecting the presence of a MLANA gene, wherein the presence of MLANA gene is indicative of melanoma and benign nevus, and the absence of MLANA gene is indicative of other cell types.

In addition to the initial prediction of the presence or absence of melanoma in a sample of a patient, melanoma can be further differentiated from benign nevus by monitoring the presence and the absence of PHACTR1 gene and SPP1 gene, optionally with MLANA gene serving as positive control.

Disclosed herein is a method for differentiating melanoma from benign nevus in a human subject, wherein the presence of either PHACTR1 or SPP1 gene is indicative of melanoma in said human subject, and the absence of both PHACTR1 and SPP1 gene is indicative of benign nevus in said human subject.

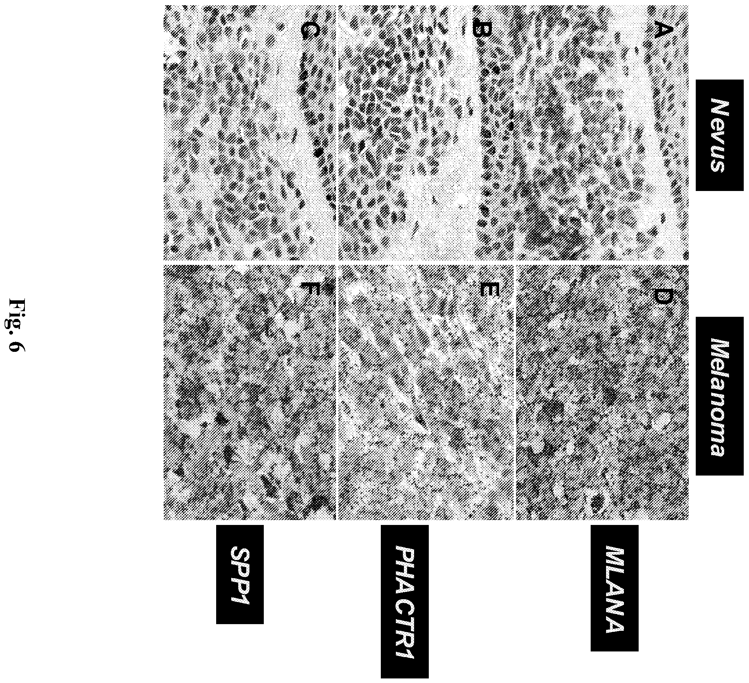

In one instance illustrated in **Fig. 6****,** skin samples of a human subject with benign nevus and skin samples of human subject with melanoma were tested for the presence of marker genes PHACTR1, SPP1 and MLANA. In skin sample of presented in Figs. 6A, 6B and 6C, the sample is completely negative for PHACTR1 and SPP1, and positive for MLANA, demonstrating that the sample is absent of melanoma cells but have melanocytes and thus has benign nevus. In the sample presented in Figs. 6D, 6E and 6F, the sample is positive for all three genes: PHACTR1, SPP1 and MLANA, indicating it has melanoma, which is a malignant type of melanocytes.

In one instance the pattern of expression levels of marker genes PHACTR1 and SPP1 are used to diagnose the presence of melanoma or to provide prognosis of the likelihood of recurrence of melanoma. In one embodiment, the levels of expression of PHACTR1 and SPP1 are determined individually, *e.g.*, using distinctive labels for each gene. The pattern of levels of expression of PHACTR1 and SPP1 genes are then analyzed to determine the presence or absence of melanoma in the tested sample. In another embodiment, levels of expression of PHACTR1 and SPP1 genes are given different weights based on their degree of influence in determining the presence of absence of melanoma. In another embodiment, the levels of expression of PHACTR1 and SPP1 are pooled and determined altogether, e.g., using an identical label for all genes. The combined expression level is then analyzed to determine the presence or absence of melanoma in the tested sample.

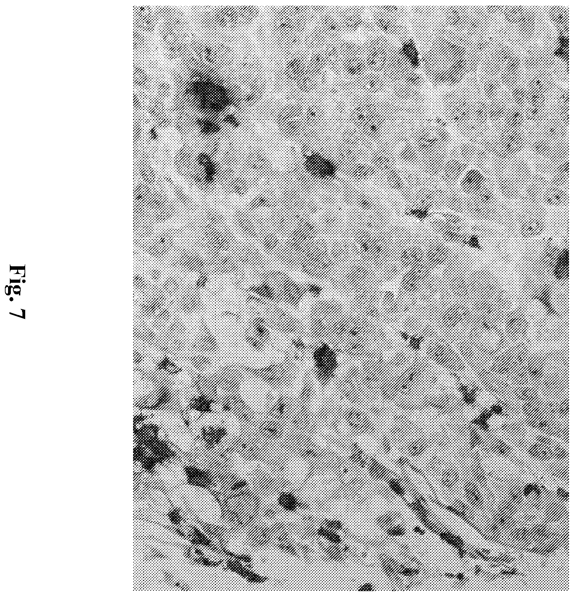

The present invention additionally identified two types of SPP1 staining pattern. In one type of SPP1 staining pattern, as shown in **Fig. 7**, the staining of SPP1 was detected predominantly in tumor-associated macrophages (TAMs), especially at the interface between tumor and stroma. Another type of SPP1 staining pattern is shown in **Fig. 6F** (SPP1 in melanoma). The staining is in both TAMs and tumor cells, and thus appears scattered around in the sample.

### 2.3 Selection of PRAME, GDF15, CXCL10, S100A6 and S100B Genes as Additional Biomarkers for Improved Detection of Melanoma

As mentioned in Section 2.1 above, it was found that a combination of PHACTR1 and SPP1 genes can detect 95 % of the melanomas. However, PHACTR1 an SPP1 genes are negative for the remaining 5% of the melanomas. Further, samples detected positive for PHACTR1 and SPP1 genes must also be positive for the expression of MLANA gene as the later is used a positive control for the detection.

Desmoplastic melanoma represents 2-3% of all melanoma. However, desmoplastic melanoma is negative in expression of MLANA. Therefore, in order to improve the scope of the melanoma detection, identification of additional marker genes which can be used to improve the scope of the melanoma detection was performed.

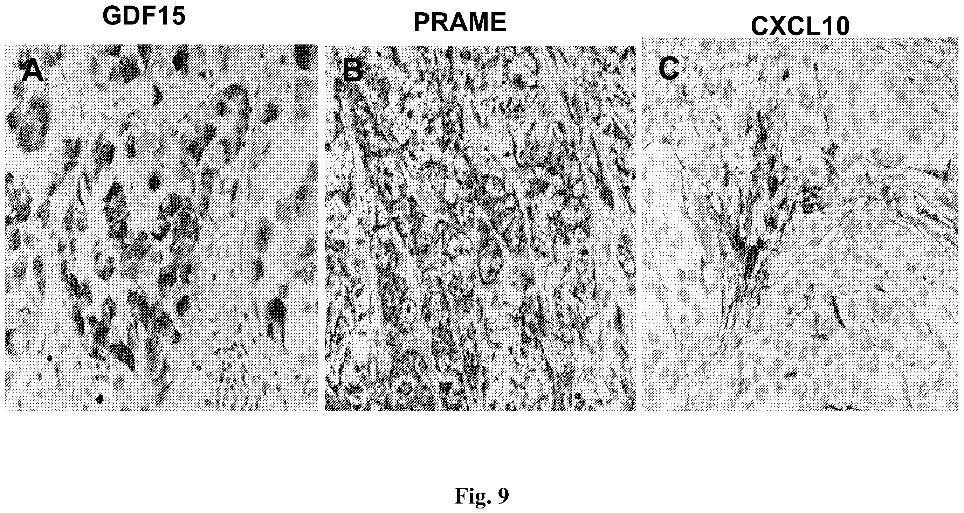

A total of 9 candidate genes (PRAME, SILV, CXCL9, CXCL10, SPRY4-IT1, GDF15, KRT15, PTN, and CFH) which are differentially expressed between melanoma and benign nevus were selected based on literature review for testing (Ugurel, S., Utikal, J. & Becker, J.C. (2009) "Tumor biomarkers in melanoma" Cancer Control: Journal of the Moffitt Cancer Center 16, 219-24; Talantov, D. et al. (2005) "Novel genes associated with malignant melanoma but not benign melanocytic lesions" Clinical Cancer Research 11, 7234-42; Koh, S.S. et al. (2009) "Molecular classification of melanomas and nevi using gene expression microarray signatures and formalin-fixed and paraffin-embedded tissue" Modern Pathology 22, 538-46; Haqq, C. et al. (2005) "The gene expression signatures of melanoma progression" Proc. Natl. Acad. Sci. U S A 102, 6092-6097; Smith, A.P., Hoek, K. & Becker, D. (2005) "Whole-genome expression profiling of the melanoma progression pathway reveals marked molecular differences between nevi/melanoma in situ and advanced-stage melanomas" Cancer Biology & Therapy 4, 1018-29; Mauerer, A. et al. (2011) "Identification of new genes associated with melanoma" Experimental Dermatology 20(6):502-7; Soikkeli, J. et al. (2007) "Systematic search for the best gene expression markers for melanoma micrometastasis detection" The Journal of Pathology 213, 180-9; Westekemper, H. et al. (2010) "Expression of MCSP and PRAME in conjunctival melanoma" British Journal of Ophthalmology 94, 1322-1327; Riker, A.I. et al. (2008) "The gene expression profiles of primary and metastatic melanoma yields a transition point of tumor progression and metastasis" BMC Medical Genomics 1, 13; Amatschek, S. et al. (2011) "CXCL9 induces chemotaxis, chemorepulsion and endothelial barrier disruption through CXCR3-mediated activation of melanoma cells" British Journal of Cancer 104, 469-79; Khaitan, D. et al. (2011) "The Melanoma-Upregulated Long Noncoding RNA SPRY4-IT1 Modulates Apoptosis and Invasion" Cancer Research 71(11):3852-62). RNAscope® assays for the 9 selected genes were performed to test their diagnostic performance in FFPE tissue microarrays of 100 samples including 77 melanoma and 23 benign nevus samples. Among the 9 candidiate genes, three genes, PRAME, GDF15, CXCL10, were selected for their superb specificity to be expressed only in melanoma but not in benign nevus. These three genes all show positive staining in melanomas and negative staining in nevi, with no false positives (**Fig. 9**, and **Table 3**). Before the present invention, there was no connection described between the expression level of a gene set consisting of PHACTR1, SPP1, PRAME, GDF15, and CXCL10 or any combination thereof and the diagnosis/prognosis of melanoma.

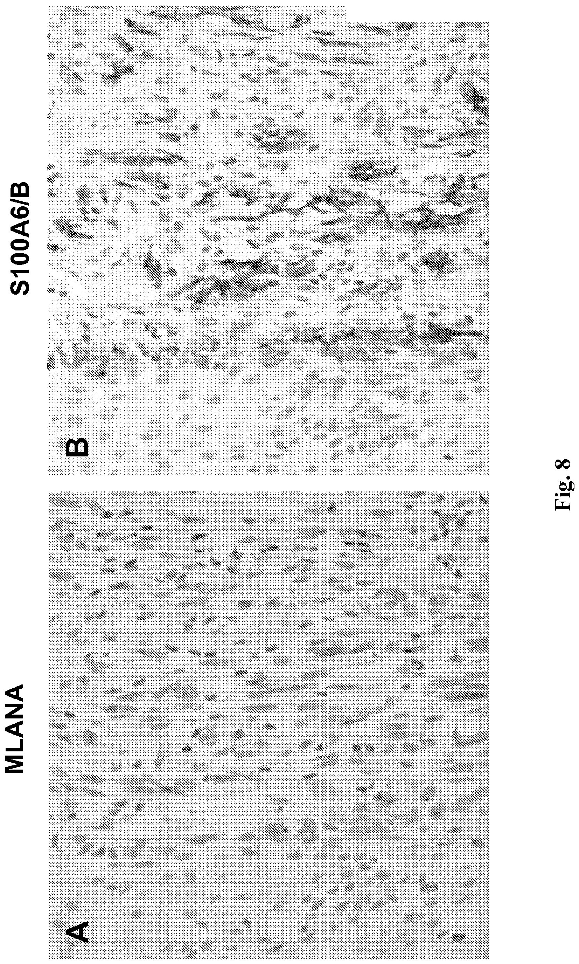

S100A6 and S100B were known for expression in desmoplastic melanoma which are normally melan-A-negative (**Fig. 8**). Thus, S100A6 and S100B genes serve as control for assay success and also as positive control for desmoplastic melanoma.

### 2.4 Detection of Melanoma Using Biomarkers of PHACTR1, SPP1, MLANA, PRAME, GDF15, CXCL10, S100A6 and/or S100B

One embodiment of the invention provides methods for detection of melanoma cells in a sample based on the level of expression of PHACTR1, SPP1, MLANA, PRAME, GDF15, CXCL10, S100A6 and S100B markers. In one embodiment, samples tested upregulated for any one of PHACTR1, SPP1, PRAME, GDF15, and CXCL10 markers are considered to have melanoma. In a specific embodiment, samples tested negative (absence) for PHACTR1, SPP1, PRAME, GDF15, and CXCL10 markers but positive (presence) of MLANA, S100A6 or S100B genes are considered to have benign nevus. Samples tested positive for any one of PHACTR1, SPP1, PRAME, GDF15, and CXCL10 markers or any combination thereof, together with positive result for any one of MLANA, S100A6 and S100B markers or any combination thereof are considered to have melanoma. MLANA, S100A6 and S100B gene serve as positive control for assay success and a marker for melanocytes. In addition, bacterial gene DapB serves as negative control.

Disclosed herein is a method for detecting melanoma in a human subject having a skin lesion suspected of melanoma, comprising the following steps:
(a) obtaining a tissue sample of said skin lesion from said human subject, wherein said tissue sample comprises a plurality of epidermal cells; and
(b) detecting the presence of a plurality of genes in said tissue sample, wherein said plurality of genes comprise PHACTR1, SPP1, PRAME, GDF15, and CXCL10 markers, wherein the presence of any one of the five gene is indicative of melanoma in said human subject.

In another embodiment, the detecting step of the above method for detecting melanoma further comprises detecting the presence of MLANA, S100A6 and S100B markers. The presence of MLANA, S100A6, or S100B markers is indicative of melanoma or and benign nevus, and the absence of these genes is indicative of other cell types.

In addition to the initial prediction of the presence of melanoma in a sample of a patient, the present invention also provides a method for differentiating melanoma from benign nevus based on the expression level of PHACTR1, SPP1, PRAME, GDF15, and CXCL10 genes, with MLANA, S100A6 and S100B genes serving as positive controls.

Disclosed herein is a method for differentiating melanoma from benign nevus in a human subject, comprising the following steps:
(a) obtaining a tissue sample of said human subject, wherein said tissue sample comprises a plurality of melanocytes; and
(b) detecting the presence of a plurality of genes in said tissue sample, wherein said plurality of genes comprise PHACTR1 marker, either alone or in combination with any one or more of SPP1, PRAME, GDF15, and CXCL10 markers,
wherein the presence of any one of the five genes is indicative of melanoma in said human subject, and the absence of both all five gene is indicative of benign nevus in said human subj ect.

In another embodiment, the detecting step of the above method for further comprising detecting the presence of MLANA, S100A6 and S100B genes. The presence of MLANA, S100A6 or S100B genes is indicative of melanoma or and benign nevus, and the absence of these genes is indicative of other cell types.

### 2.5 Measuring The Expression Levels of PHACTR1, SPP1, PRAME, GDF15, and CXCL10 Genes

The invention also concerns the determination of the expression level of PHACTR1 marker in combination with the expression level of SPP1, PRAME, GDF15, and CXCL10 markers in a tissue sample. In a preferred embodiment, the determination is made *in situ* using a gene expression analysis assay which measures mRNA in solution. According to one aspect, the sample may be a solid body sample from a mammal, especially a sample of skin. The sample may be taken and if necessary prepared according to methods known to a person skilled in the art. As will be disclosed below, in the method according to the invention, detection/determination of gene expression level may be carried out using the auxiliary methods of RNAscope®, Nanostring, QuantiGene® 2.0, qNPA™ ELISA (enzyme-linked immunosorbent assay), gene/protein microarrays, RT-PCR, sequencing, etc.These methods are known in the art, particularly in the area of medical research. Instructions for the use of some these methods and other methods not discussed here may be found *e.g.* in Sambrook, Fritsch, and Maniatis, "Molecular Cloning, A Laboratory Manual".

Disclosed herein is a method for measuring a plurality of markers in a human subject, comprising the following steps:
(a) obtaining a tissue sample from said human subject, wherein said tissue sample comprises a plurality of melanocytes; and
(b) determining the level of expression of PHACTR1, either alone or in combination with any one or more of SPP1, PRAME, GDF15, and CXCL10 genes, or fragments thereof.

### 3. Detection Methods

All aspects of this invention are generally applicable to *in situ* detection of nucleic acids in individual cells. However, many features of this invention can also be used in whole-sample nucleic acid detection applications.

In a preferred embodiment of the invention, the level of the biological marker is determined in vitro in a biological sample obtained from an individual. For in vitro determining the level of the biological markers of the present invention, any suitable biological sample from bodily skin that may comprise a biological marker identified by this research may be used. The biological sample may be processed according to well-known techniques to prepare the sample for testing.

In diagnosing or identifying a melanoma, a test sample containing at least one cell from melanoma is provided to obtain a genetic sample. The test sample may be obtained using any technique known in the art including biopsy, surgical excisions, needle biopsy, scraping, etc. From the test sample is obtained a genetic sample. The genetic sample comprises a nucleic acid, preferably RNA and/or DNA. For example, in determining the expression of marker genes one can obtain mRNA from the test sample, and the mRNA may be reverse transcribed into cDNA for further analysis. In another embodiment, the mRNA itself is used in determining the expression of marker genes. In some embodiments, the expressions level of a particular marker gene may be determined by determining the level/presence of a gene product (*e.g.*, protein) thereby eliminating the need to obtain a genetic sample from the test sample.

Once a genetic sample has been obtained, it can be analyzed for the presence or absence of particular marker genes. For detection of biological markers of the invention, gene signal detection can be made of conventional methods known in the art. including, but not limited to, RNAscope®, Nanostring, QuantiGene® 2.0, qNPA™, sequencing, PCR, RT-PCR, quantitative PCR, restriction fragment length polymorphism, hybridization techniques, Northern blot, microarray technology, DNA microarray technology, etc. In determining the expression level of a marker gene or genes in a genetic sample, the level of expression may be normalized by comparison to the expression of another gene such as a well known, well characterized gene or a housekeeping gene.

The expression data from a particular marker gene or group of marker genes may be analyzed using statistical methods known in the art in order to determine the phenotype or characteristic of a particular tumor or cancer. Methods used in classifying tumors include, but not limited to: decision trees, support vector machine (SVM), neural network, etc.

### 3.1 Nucleic Acid Detection Methods

When the biological marker is a gene, and/or a polynucleotide fragment and/or variant thereof, *e.g.* DNA, cDNA, RNA, mRNA etc., such as a gene coding for a specific protein, or mRNA that is transcribed, the biological marker can be measured in *e.g.* skin biopsies, by *e.g.* well-known molecular biological assays, such as *in situ* hybridization techniques using probes directed to the specific polynucleotides. In one embodiment, the RNAscope® technology may be used to determine the gene expression *in situ* in FFPE tissue sections of melanocytic lesions. Other nucleic-acid based assays which may be used according to the invention include RT-PCR, nanostring, sequencing, nucleic acid based ELISA, Northern blotting etc, and any combinations thereof.

Generally, a method for detecting a nucleic acid marker comprises hybridizing an oligonucleotide to a marker within the nucleic acid target in a sample from a subject under moderate to high stringency conditions and detecting hybridization of the oligonucleotide using a detection means, such as for example, an amplification reaction or a hybridization reaction or a combination of both.

In another embodiment, a marker that is associated with melanoma occurs within a protein coding region of a genomic gene (*e.g*. PHACTR1, SPP1, PRAME, GDF15, CXCL10, etc.) and is detectable in mRNA encoded by that gene. Such a marker may be detected using, for example, RNAscope®, nanostring, reverse-transcriptase PCR (RT-PCR), sequencing, transcription mediated amplification (TMA) or nucleic acid sequence based amplification (NASBA), although any mRNA or cDNA based hybridization and/or amplification protocol is clearly amenable to the instant invention.

### 3.1.1 Nucleic Acid Detection Method Based on RNAscope®

The inventors of this application have developed an *in situ* hybridization method see (United States Patent Application No. 7,709,198) called RNAscope®, that allows for the direct visualization of RNA *in situ.* This method utilizes the oligonucleotide probe sets and novel signal amplification systems described below. The assay can be used on a variety of sample types including cultured cells, peripheral blood mononuclear cells (PBMCs), frozen tissue, and formalin-fixed paraffin embedded (FFPE) tissue. In addition, the assay can utilize both chromogenic and fluorescent detection reagents.

One embodiment of this invention concerns the adaptation of RNAscope® assay technology to detect, *in situ,* marker genes of melanoma in skin sample of a patient.

### 3.1.1.1 RNAscope® Assay Technology

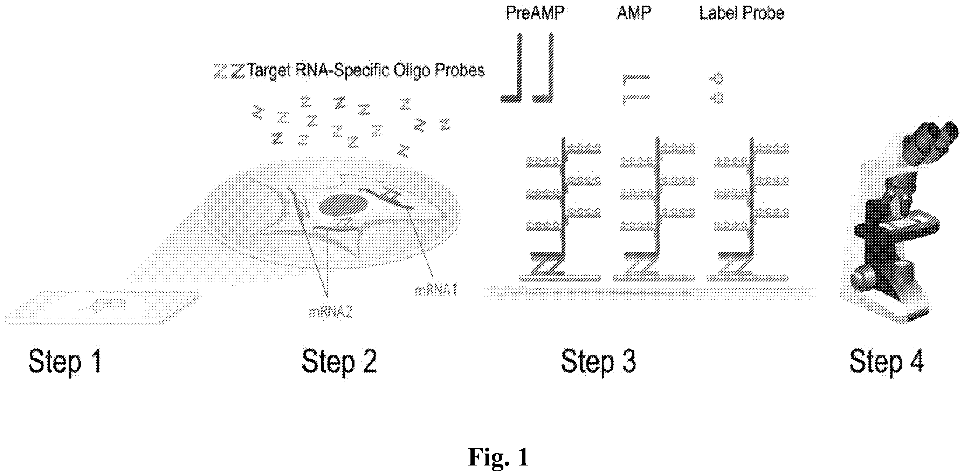

The RNAscope® assay technology provides multiplex nucleic acid assays in single cells (see **Fig. 1**). At the core of this technology is the "double Z" probe design, which allows robust amplification of specific hybridization signals without also amplifying nonspecific events. Each target probe ("Z") has a target-specific sequence, which binds to the target mRNA, a spacer, and a "tail" sequence. Two target probes (double Z) hybridize contiguously onto a target mRNA, and the two "tail" sequences form a 28-base hybridization site for the PreAmplifier. The double Z probe design ensures high fidelity of signal amplification because 1) it is highly unlikely that a pair of target probes will hybridize nonspecifically juxtaposed to each other to form a binding site for the PreAmplifier; and 2) neither tail alone can bind efficiently to the PreAmplifier under the assay conditions. The PreAmplifier, Amplifier and Label Probe are hybridized sequentially to each target probe pair, resulting in the accumulation of as many as 8,000 label molecules per 1 kb of target RNA. The Label Probe can be conjugated to either a fluorophore or a chromogenic enzyme (*e.g*., HRP), enabling viewing of hybridization signals under a standard bright-field or epifluorescent microscope, respectively. With a fluorescent Label Probe, the signals can contain at least 100-fold more fluorescent molecules than traditional RNA fluorescent ISH methods and are readily visible under a standard fluorescent microscope.

In addition, multiple signal amplifiers have been built that each recognizes a unique tail sequence on the target probes, allowing for the simultaneous visualization of multiple target RNAs. Importantly, this assay is compatible with partially degraded RNA present in archival FFPE tissues, since the double Z probe pairs target short regions of ∼50 nucleotides in length.

### 3.1.1.2 Single Molecule Detection by RNAscope®

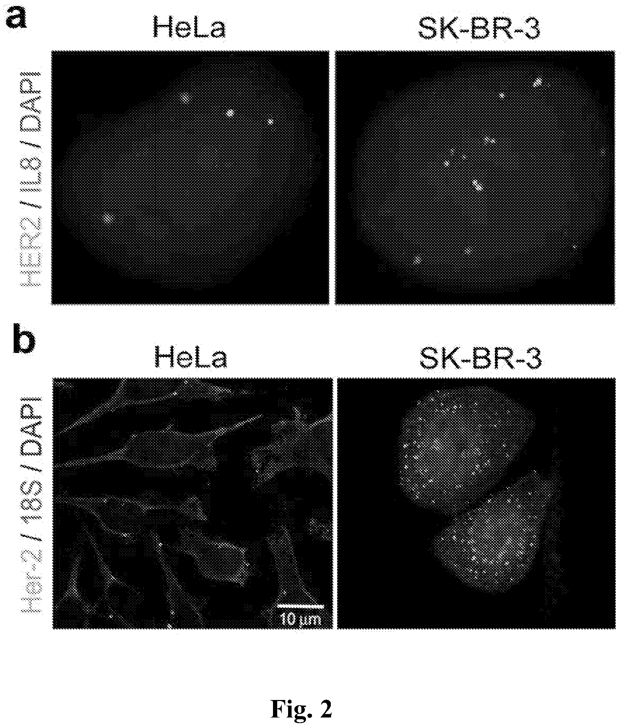

The marked improvement in signal-to-noise ratio with RNAscope® allows detection of single RNA molecules as punctuate dots on the stained slides. To demonstrate this, a probe set was used to detect human epidermal growth factor receptor 2 (HER2) mRNA and genomic DNA in SK-BR-3 and HeLa cells. For genomic DNA, two fluorescent dots were detected in HeLa cells whereas many more dots were seen in SK-BR-3 cells, which is consistent with the HER2 gene amplification present in this cell line (**Fig. 2a**). The same probe set was used to detect HER2 mRNA transcripts, and the expression levels in HeLa and SK-BR-3 cells were consistent with their gene amplification status (**Fig. 2b**). These results demonstrate that RNAscope® is capable of single RNA molecule detection. Furthermore, the number of HER2 mRNA dots in HeLa cells were counted and compared that with the number of mRNA transcripts determined by QuantiGene® 2.0, a solution-based assay that can measure RNA transcripts quantitatively. The average copy number per cell determined by RNAscope® was comparable to that determined by QuantiGene® 2.0, again indicating that each dot is likely to represent a single mRNA molecule.

### 3.1.1.3 Detection of mRNA in FFPE tumor tissues by RNAscope®

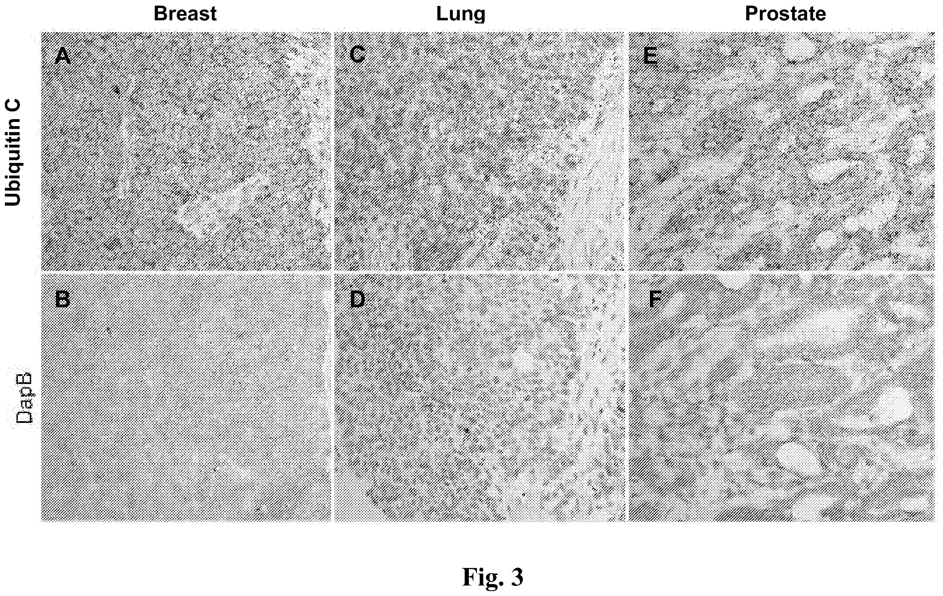

Since each double Z probe pair targets a region of ∼50 bases in length, RNAscope® has built-in robustness against partial RNA degradation, as is often the case in archival FFPE tissue. This was demonstrated by hybridizing probe sets targeting the housekeeping gene ubiquitin C (UBC) to archival breast, lung and prostate tissues following standard tissue pretreatment in boiling citrate buffer and protease digestion. Strong positive staining was detected in all three tissue types, but no signal was present when the negative control DapB probes were used **(****Fig. 3****)**.

### 3.1.1.4 Using RNAscope® for Detection of Melanoma

In one example, RNAscope® was used to detect three nucleic acid targets PHACTR1, SPP1, and MLANA in a melanocyte of a patient. In the methods, a sample comprising the cell is provided. The cell tested comprises, or is suspected of comprising, PHACTR1, SPP1, and MLANA. Provided in the assay are: a first label probe comprising a first label, a second label probe comprising a second label, and a third label probe comprising a third label, wherein the signals from the three labels are distinguishable from each other. Alternatively, PHACTR1 and SPP1 labels are identical and the signals emitted from the two markers are indistinguishable from each other. For each of the three nucleic acid targets PHACTR1, SPP1, and MLANA, three target probe sets, each comprising at least two target probes are provided.

In one instance, the first nucleic acid target is PHACTR1, the second nucleic acid target is SPP1 and the third nucleic acid target is MLANA. When identical labels are used for PHACTR1 and SPP1, detection of label signals in a sample indicates that either PHACTR1 or SPP1, or both existed in the sample. The detection of uniformed label signals suggests the presence of melanoma in the sample. When the three labels are distinguishable, detection of each individual labels provides further information regarding the expression of the individual genes in the sample.

The first target probe set is hybridized, in the cell, to the first nucleic acid target PHACTR1 (when the first nucleic acid target is present in the cell), the second target probe set is hybridized, in the cell, to the second nucleic acid target SPP1 (when the second nucleic acid target is present in the cell), and the third target probe set is hybridized, in the cell, to the third nucleic acid target MLANA (when the third nucleic acid target is present in the cell). The first label probe is captured to the first target probe set, the second label probe is captured to the second target probe set, and the third label probe is captured to the third target probe set, thereby capturing the first label probe to the first nucleic acid target PHACTR1, the second label probe to the second nucleic acid target SPP1 and the third label probe to the third nucleic acid target MLANA. The first signal from the first label, the second signal from the second label and the third signal from the third label are then detected. Since the first, second and third labels are associated with their respective nucleic acid targets through the target probes, presence of the label(s) in the cell indicates the presence of the corresponding nucleic acid target(s) in the cell. The methods are optionally quantitative. Thus, an intensity of the first, second and third signal can be measured, and the intensity of the first signal can be correlated with a quantity of PHACTR1, SPP1 and MLANA in the cell. As another example, a signal spot can be counted for each copy of the PHACTR1, SPP1 and MLANA genes to quantitate them,.

In one embodiment, the label probes bind directly to the target probes. In another embodiment the label probes are captured to the target probes indirectly, for example, through binding of preamplifiers and/or amplifiers. Use of amplifiers and preamplifiers can be advantageous in increasing signal strength, since they can facilitate binding of large numbers of label probes to each nucleic acid target.

In the above classes of embodiments, one target probe hybridizes to each label probe, amplifier, or preamplifier. In alternative classes of related embodiments, two or more target probes hybridize to the label probe, amplifier, or preamplifier.

In embodiments in which two or more target probes are employed, the target probes preferably hybridize to nonoverlapping polynucleotide sequences in their respective nucleic acid target. The target probes can, but need not, cover a contiguous region of the nucleic acid target. Blocking probes, polynucleotides which hybridize to regions of the nucleic acid target not occupied by target probes, are optionally provided and hybridized to the target. For a given nucleic acid target, the corresponding target probes and blocking probes are preferably complementary to physically distinct, nonoverlapping sequences in the nucleic acid target, which nonoverlapping sequences are preferably, but not necessarily, contiguous. Having the target probes and optional blocking probes be contiguous with each other can in some embodiments enhance hybridization strength, remove secondary structure, and ensure more consistent and reproducible signal.

As noted, the methods are useful for multiplex detection of nucleic acids, including simultaneous detection of more than three nucleic acid targets. Thus, the cell optionally comprises or is suspected of comprising a fourth, fifth, sixth, seventh or even more nucleic acid target. The method that detects a fourth nucleic acid target comprises: providing a fourth label probe comprising a fourth label, wherein a fourth signal from the fourth label can be either distinguishable or indistinguishable from the first three signals depending on the degree of information to be collected, providing at least two fourth target probes, hybridizing in the cell the fourth target probe to the fourth nucleic acid target (when the fourth target is present in the cell), capturing the fourth label probe to the fourth target probe, and detecting the fourth signal from the fourth label. As disclosed herein, the additional nucleic acid targets include but are not limited to PRAME, GDF15, CXCL10, S100A6, S100B, etc.

In detection of nucleic acid targets in a cell, the cell is typically fixed and permeabilized before hybridization of the target probes, to retain the nucleic acid targets in the cell and to permit the target probes, label probes, etc. to enter the cell. The cell is optionally washed to remove materials not captured to one of the nucleic acid targets. The cell can be washed after any of various steps, for example, after hybridization of the target probes to the nucleic acid targets to remove unbound target probes, after hybridization of the preamplifiers, amplifiers, and/or label probes to the target probes, and/or the like.

The various capture and hybridization steps can be performed simultaneously or sequentially, in essentially any convenient order. Preferably, a given hybridization step is accomplished for all of the nucleic acid targets at the same time. For example, all the target probes (first, second, etc.) can be added to the cell at once and permitted to hybridize to their corresponding targets, the cell can be washed, amplifiers (first, second, etc.) can be hybridized to the corresponding target probes, the cell can be washed, the label probes (first, second, etc.) can be hybridized to the corresponding amplifiers, and the cell can then be washed again prior to detection of the labels. As another example, the target probes can be hybridized to the targets, the cell can be washed, amplifiers and label probes can be added together and hybridized, and the cell can then be washed prior to detection. It will be evident that double-stranded nucleic acid target(s) are preferably denatured, e.g., by heat, prior to hybridization of the corresponding target probe(s) to the target(s).

In some embodiments, the cell from a tissue sample is in suspension for all or most of the steps of the method, for ease of handling. However, the methods are also applicable to cells in solid tissue samples (*e.g.,* tissue sections) and/or cells immobilized on a substrate (*e.g.,* a slide or other surface). Thus, in one class of embodiments, the cell is in suspension in the sample comprising the cell, and/or the cell is in suspension during the hybridizing, capturing, and/or detecting steps. For example, the cell can be in suspension in the sample and during the hybridization, capture, optional washing, and detection steps. In other embodiments, the cell is in suspension in the sample comprising the cell, and the cell is fixed on a substrate during the hybridizing, capturing, and/or detecting steps. For example, the cell can be in suspension during the hybridization, capture, and optional washing steps and immobilized on a substrate during the detection step. In other embodiments, the sample comprises a tissue section.

### 3.1.2 Additional Nucleic Acid Detection Methods

RT-PCR. Methods of RT-PCR are known in the art and described, for example, in Dieffenbach (Ed) and Dveksler (Ed) (In: PCR Primer: A Laboratory Manual, Cold Spring Harbor Laboratories, NY, 1995).

Nanostring. Methods of Nanostring use labeled reporter molecules, referred to as labeled "nanoreporters," that are capable of binding individual target molecules. Through the nanoreporters' label codes, the binding of the nanoreporters to target molecules results in the identification of the target molecules. Methods of Nanostring are described in U.S. Patent No. 7,473,767.

Sequencing. The nucleic acid molecules can also be sequenced by any method known in the art, e.g., ensemble sequencing or single molecule sequencing. One conventional method to perform sequencing is by chain termination and gel separation, as described by Sanger et al., Proc Natl Acad Sci U S A, 74(12): 5463 67 (1977). Another conventional sequencing method involves chemical degradation of nucleic acid fragments. See, Maxam et al., Proc. Natl. Acad. Sci., 74: 560 564 (1977). Finally, methods have been developed based upon sequencing by hybridization. See, *e.g*., Drmanac, et al. (Nature Biotech., 16: 54 58, 1998).

qNPA™. The quantitative nuclease protection assay (qNPA™) is a method for measuring gene expression levels (mRNA) using gene-specific DNA oligonucleotides. S1 nuclease is used to degrade any non-hybridized nucleic acid, such as the non-hybridized portion of the targeted RNA, all of the non-targeted RNA, and excess DNA oligonucleotides. The resulting DNA oligos are a stoichiometrically representative library of the original RNA sample. The individual DNA probe oligonucleotides are present in the precise relative abundance as the RNA transcripts were in the original sample. Methods of qNPA™ are described at its manufacture's website: http://(www).htgmolecular.com.

QuantiGene® 2.0. The QuantiGene® 2.0 assay uses branched DNA (bDNA) technology. The bDNA assay is a sandwich nucleic acid hybridization method that uses bDNA molecules to amplify signal from captured target RNA. RNA is measured directly from the sample source in bDNA assays. Methods of QuantiGene® 2.0 assays are described at its manufacture's website: http://(www).panomics.com.

### 3.2 Protein Detection Methods

When the biological marker is a protein and/or a fragment and/or a variant thereof, several conventional methods for determining the level of a specific protein, and/or fragments and/or variants thereof, which are well-known to the skilled person, may be used. The level of the marker may for example be measured by using immunological assays, such as enzyme-linked immunosorbent assays (ELISA), thus providing a simple, reproducible and reliable method. Antibodies for use in such assays are available, and additional (polyclonal and monoclonal) antibodies may be developed using well-known standard techniques for developing antibodies. Other methods for measuring the level of the biological protein markers may furthermore include (immuno)histochemistry, Western blotting, flow-cytometry, RIA, competition assays, etc. and any combinations thereof.

### 3.2.1 Ligands and Antibodies

It will be apparent to the skilled artisan based on the disclosure herein that the present invention also extends to detection of a marker in a polypeptide, *e.g*., a target polypeptide encoded by a PHACTR1 mRNA. It is clear to a skilled artisan that detection of other target polypeptides such as SPP1, MLANA, PRAME, GDF15, CXCL10, S100A6, and S100B can be conducted in the same way as described below for PHACTR1.

Methods for detecting target polypeptides generally make use of a ligand or antibody that preferentially or specifically binds to the target polypeptide. As used herein the term "ligand" shall be taken in its broadest context to include any chemical compound, polynucleotide, peptide, protein, lipid, carbohydrate, small molecule, natural product, polymer, etc. that is capable of selectively binding, whether covalently or not, to one or more specific sites on an PHACTR1 polypeptide. The ligand may bind to its target via any means including hydrophobic interactions, hydrogen bonding, electrostatic interactions, van der Waals interactions, pi stacking, covalent bonding, or magnetic interactions amongst others.

As used herein, the term "antibody" refers to intact monoclonal or polyclonal antibodies, immunoglobulin (IgA, IgD, IgG, IgM, IgE) fractions, humanized antibodies, or recombinant single chain antibodies, as well as fragments thereof, such as, for example Fab, F(ab)2, and Fv fragments.

It will be apparent to the skilled artisan that the ligand and antibody against all the markers disclosed in the present invention can be prepared in the way as described below.

Antibodies are prepared by any of a variety of techniques known to those of ordinary skill in the art, and described, for example in, Harlow and Lane (In: Antibodies: A Laboratory Manual, Cold Spring Harbor Laboratory, 1988). In one such technique, an immunogen comprising the antigenic polypeptide is initially injected into any one of a wide variety of animals (e.g., mice, rats, rabbits, sheep, humans, dogs, pigs, chickens and goats). The immunogen is derived from a natural source, produced by recombinant expression means, or artificially generated, such as by chemical synthesis (*e.g.*, BOC chemistry or FMOC chemistry).

A peptide, polypeptide or protein is joined to a carrier protein, such as bovine serum albumin or keyhole limpet hemocyanin. The immunogen and optionally a carrier for the protein is injected into the animal host, preferably according to a predetermined schedule incorporating one or more booster immunizations, and blood collected from said the animals periodically. Optionally, the immunogen is injected in the presence of an adjuvant, such as, for example Freund's complete or incomplete adjuvant, lysolecithin and dinitrophenol to enhance the subject's immune response to the immunogen. Monoclonal or polyclonal antibodies specific for the polypeptide are then purified from blood isolated from an animal by, for example, affinity chromatography using the polypeptide coupled to a suitable solid support.

Monoclonal antibodies specific for the antigenic polypeptide of interest are prepared, for example, using the technique of Kohler and Milstein, Eur. J. Immunol. 6:511-519, 1976, and improvements thereto. Briefly, these methods involve the preparation of immortal cell lines capable of producing antibodies having the desired specificity (i.e., reactivity with the polypeptide of interest). Such cell lines are produced, for example, from spleen cells obtained from an animal immunized as described supra. The spleen cells are immortalized by, for example, fusion with a myeloma cell fusion partner, preferably one that is syngenic with the immunized animal. A variety of fusion techniques are known in the art, for example, the spleen cells and myeloma cells are combined with a nonionic detergent or electrofused and then grown in a selective medium that supports the growth of hybrid cells, but not myeloma cells. A preferred selection technique uses HAT (hypoxanthine, aminopterin, and thymidine) selection. After a sufficient time, usually about 1 to 2 weeks, colonies of hybrids are observed. Single colonies are selected and growth media in which the cells have been grown is tested for the presence of an antibody having binding activity against the polypeptide (immunogen). Hybridomas having high reactivity and specificity are preferred.

It is preferable that an immunogen used in the production of an antibody is one which is sufficiently antigenic to stimulate the production of antibodies that will bind to the immunogen and is preferably, a high titer antibody. In one embodiment, an immunogen is an entire protein.

In another embodiment, an immunogen consists of a peptide representing a fragment of a polypeptide, for example a region of a PHACTR1 polypeptide. Preferably an antibody raised to such an immunogen also recognizes the full-length protein from which the immunogen was derived, such as, for example, in its native state or having native conformation.

Alternatively, or in addition, an antibody raised against a peptide immunogen recognizes the full-length protein from which the immunogen was derived when the protein is denatured. By "denatured" is meant that conformational epitopes of the protein are disrupted under conditions that retain linear B cell epitopes of the protein. As will be known to a skilled artisan linear epitopes and conformational epitopes may overlap.

Alternatively, a monoclonal antibody capable of binding to a form of a PHACTR1 polypeptide or a fragment thereof is produced using a method such as, for example, a human B-cell hybridoma technique (Kozbar et al., Immunol. Today 4:72, 1983), a EBV-hybridoma technique to produce human monoclonal antibodies (Cole et al. "Monoclonal Antibodies in Cancer Therapy", 1985 Allen R. Bliss, Inc., pages 77-96), or screening of combinatorial antibody libraries (Huse et al., Science 246:1275, 1989).

Such an antibody is then particularly useful in detecting the presence of a marker of melanoma.

The methods described supra are also suitable for production of an antibody or antibody binding fragment as described herein according to any embodiment.

### 3.2.2 Detection Methods

In one embodiment, the method of the invention detects the presence of a marker in a polypeptide, aid marker being associated with melanoma.

An amount, level or presence of a polypeptide is determined using any of a variety of techniques known to the skilled artisan such as, for example, a technique selected from the group consisting of, immunohistochemistry, immunofluorescence, an immunoblot, a Western blot, a dot blot, an enzyme linked immunosorbent assay (ELISA), radioimmunoassay (RIA), enzyme immunoassay, fluorescence resonance energy transfer (FRET), matrix-assisted laser desorption/ionization time of flight (MALDI-TOF), electrospray ionization (ESI), mass spectrometry (including tandem mass spectrometry, e.g. LC MS/MS), biosensor technology, evanescent fiber-optics technology or protein chip technology.

In one example, an assay used to determine the amount or level of a protein is a semi-quantitative assay. In another example, an assay used to determine the amount or level of a protein in a quantitative assay.

Preferably, an amount of antibody or ligand bound to a marker of a melanoma in a PHACTR1 polypeptide is determined using an immunoassay. Preferably, using an assay selected from the group consisting of, immunohistochemistry, immunofluorescence, enzyme linked immunosorbent assay (ELISA), fluorescence linked immunosorbent assay (FLISA) Western blotting, RIA, a biosensor assay, a protein chip assay, a mass spectrometry assay, a fluorescence resonance energy transfer assay and an immunostaining assay (*e.g.* immunofluorescence).

### 4. Detection Kit

The present invention further relates to kits for performing the diagnostic methods as described above. The invention in particular relates to such diagnostic kits for identifying a subject with melanoma, comprising means for receiving one or more biological samples of said subject, and means for determining the level of the biological marker(s) in said biological sample of said subject. Thus a kit is provided which can be used as a reliable and easy diagnostic tool. One exemplary means for receiving the biological sample comprises a FFPE. The means for determining the presence of PHACTR1, SPP1, PRAME, GDF15, and CXCL10 genes in said biological sample of said subject may for example comprise one or more specific polynucleotide probes, antibodies, primers, etc. suitable for detecting the biological marker(s), identified according to the present invention. One exemplary means for determining the presence of the marker genes is an RNAscope® assay kit equipped with mRNA markers which encode fragments of PHACTR1, SPP1, PRAME, GDF15, and CXCL10 genes, respectively. Another means for determining the presence of the marker genes is an RNAscope® assay kit equipped with mRNA markers which encode fragments of PHACTR1 and SPP1. The kits may further also comprise calibration means and instruction for use.

One general class of embodiments of the present invention provides a kit for detecting a nucleic acid target genes of PHACTR1, SPP1, PRAME, GDF15, and CXCL10 in an individual cell. One embodiment provides a kit for detecting a nucleic acid target genes of PHACTR1 and SPP1. The kits comprise one or more tissue pretreatment reagents, a target probe set, and a signal amplification system, packaged in one or more containers. The tissue pretreatment reagents comprise a citrate buffer and a protease. Tissue pretreatment procedure comprises heating the FFPE tissue sections in a citrate buffer followed by digestion with a protease. The purpose of these treatments is to allow for RNA access by the target probes through the disruption of formaldehyde cross-linking created in the sample preparation procedure. The target probe are designed to hybridize to each of the marker genes. In addition, probes against MLANA, S100A6 and S100B may be also included in the kit as positive controls, and a probe against the bacterial gene DapB maye be included in the kit as negative control. The signal amplification system comprises, as described above in Section 3.1.1.4, pre-amplifier, amplifier, label probe and label. Each probe set contains a recognition sequence for a signal amplification system. The label probe refers to an entity that binds to the target molecule, directly or indirectly, and enables the target to be detected by a readout instrument. In one preferred embodiment, the label is Fast Red. Nucleic acid targets such as PHACTR1, SPP1, PRAME, GDF15, and CXCL10 can be detected by the formation of a precipitate following incubation with Fast Red.

Essentially all of the features noted for the embodiments in Sections 2.2 and 2.4 above apply to the kits as well, as relevant; for example, with respect to the identity of the markers, the expression level of the markers, the upregulation of the gene expression level or protein expression level of the markers, the patterns of the expression levels, and the type of gene expression analysis assays.

The features noted for the embodiments in Section 2.1.1.4 above apply to the kits as well, as relevant; for example, with respect to number of nucleic acid targets, configuration and number of the label and target probes, inclusion of preamplifiers and/or amplifiers, inclusion of blocking probes, inclusion of amplification reagents, type of nucleic acid target, location of various targets on a single molecule or on different molecules, type of labels, inclusion of optional blocking probes, and/or the like. The kit optionally also includes instructions for detecting the nucleic acid targets in the cell and/or identifying the cell as being of a specified type, one or more buffered solutions (*e.g*., diluent, hybridization buffer, and/or wash buffer), reference cell(s) comprising one or more of the nucleic acid targets, and/or the like.

One embodiment, the present invention provides a kit for detecting the expression level of PHACTR1 marker in combination with SPP1, PRAME, GDF15, CXCL10 and optionally MLANA, S110A, and S100B markers in an tissue sample, with all of the features noted for the embodiments above apply to this kit as well.

### 5. Prognosis of Recurrence of Melanoma

In addition to the initial detection of melanoma in a sample of a patient, the present invention can be used for prognosis of the likelihood of recurrence of melanoma in patients.

As disclosed above, the present invention uses a set of PRAME and SPP1 markers or a set of PHACTR1, SPP1, MLANA, PRAME, GDF15, and CXCL10 markers to determine the presence of melanoma. It is also discovered in the present invention that the same sets of marker genes can be used to predict the likelihood of recurrence of melanoma in patients.

In one embodiment, provided herein is a method of providing a prognosis to a human subject who is at risk of developing melanoma recurrence, comprising the following steps:
(a) obtaining a tissue sample from said human subject, wherein said tissue sample comprises a plurality of melanocytes;
(b) measuring the level of expression of PRAME and SPP1 marker and optionally the level of expression of PHACTR1, GDF15, and CXCL10 markers; and
based on the gene expression levels of the PRAME and SPP1 marker and the additional markers obtained for the tissue sample, providing a prognosis to the subject.

In another instance, level of expression of only PHACTR1 and SPP1 markers are used in the method for providing prognosis of melanoma.

All of the features noted for the embodiments described above in Sections 2.2 and 2.4 apply to the prognosis of recurrence of melanoma as well.

### 6. Determining the Stage of Melanoma Based on Sentinel Lymph Node

In addition to the diagnosis and prognosis of melanoma in a sample of a patient, the methods discloses herein can be used to determine the stage of the melanoma patients. Currently, the best single prognostic factor for stage I-II disease is sentinel lymph node (SLN) status (Balch, C.M. et al. (2004) "An Evidence-based Staging System for Cutaneous Melanoma". CA: A Cancer Journal for Clinicians 54, 131-149). Positive SLN status indicates presence of micrometastasis and portends future metastasis. However, the standard procedure of SLN assessment is based on histopathology, resulting in underestimating micrometastasis by 10-20%. RT-PCR has been proposed as a tool for molecular staging, but it uses markers not distinguishing between benign and malignant melanocytes and may not work well when there are only a few tumor cells present (Soikkeli, J. et al. (2007) "Systematic search for the best gene expression markers for melanoma micrometastasis detection" The Journal of Pathology 213, 180-9; Mocellin, S., Hoon, D.S.B., Pilati, P., Rossi, C.R. & Nitti, D. (2007) "Sentinel lymph node molecular ultrastaging in patients with melanoma: a systematic review and meta-analysis of prognosis" Journal of Clinical Oncology 25, 1588-95; Taback, B. et al. (2001) "The clinical utility of multimarker RT-PCR in the detection of occult metastasis in patients with melanoma" Recent Results in Cancer Research. 158:78-92).

As disclosed above, the present invention can detect positive staining in a few cells with and thus with high sensitivity. It can also differentiate melanoma with benign nevus with 100% accuracy. Further, the staining pattern of SPP1 can interrogate tumor morphology in both tumor and its microenvironment. Thus, in one example of the invention, it has been surprisingly found that the biomarkers claimed in the present invention have the potential for prognosis of newly diagnosed melanoma patients by testing the patient's sentinel lymph node.

Disclosed herein is a method for determining the stage of melanoma in a human subject, comprising the following steps:
obtaining a tissue sample from said human subject, wherein said tissue sample comprises sentinel lymph node;
determining the level of expression of PHACTR1 marker and optionally the level of expression of one or more SPP1, PRAME, GDF15, and CXCL10 markers;
thereby determining the stage of melanoma as characterized by the presence of malignant melanoma cells in sentinel lymph node, based on the expression levels in said tissue sample.

In another instance, level of expression of only PHACTR1 and SPP1 markers are used in the method for determining the stage of melanoma.

All of the features noted for the embodiments described above in Sections 2.2 and 2.4 apply to the method for determining the stage of melanoma as well.

### REFERENCES:

1. Jemal, A., Siegel, R., Xu, J. & Ward, E. (2010) Cancer Statistics, CA Cancer J Clin. 60(5):277-300
2. Miller, A.J. & Mihm, M.C. Melanoma. (2006) The New England journal of medicine 355, 51-65.
3. Urso, C., Saieva, C., Borgognoni, L., Tinacci, G. & Zini, E. (2008) Sensitivity and specificity of histological criteria in the diagnosis of conventional cutaneous melanoma. Melanoma research 18, 253-8.
4. Troxel, D.B. Medicolegal (2006) Aspects of error in pathology. Archives of pathology & laboratory medicine 130, 617-9.
5. Veenhuizen, K.C. et al. (1997) Quality assessment by expert opinion in melanoma pathology: experience of the pathology panel of the Dutch Melanoma Working Party. The Journal of pathology 182, 266-72.
6. Shoo, B.A., Sagebiel, R.W. & Kashani-Sabet, M. (2010) Discordance in the histopathologic diagnosis of melanoma at a melanoma referral center. Journal of the American Academy of Dermatology 62, 751-6.
7. Welch, H.G. & Black, W.C. (2010) Overdiagnosis in cancer. Journal of the National Cancer Institute 102, 605-13.
8. Glusac, E.J. (2011) The melanoma "epidemic", a dermatopathologist's perspective. Journal of cutaneous pathology 38, 264-7.
9. Ugurel, S., Utikal, J. & Becker, J.C. (2009) Tumor biomarkers in melanoma. Cancer control: journal of the Moffitt Cancer Center 16, 219-24.
10. Prieto, V.G. & Shea, C.R. (2008) Use of immunohistochemistry in melanocytic lesions. Journal of cutaneous pathology 35 Suppl 2, 1-10.
11. Bastian, B.C., Olshen, A.B., LeBoit, P.E. & Pinkel, D. (2003) Classifying melanocytic tumors based on DNA copy number changes. The American journal of pathology 163, 1765-70.
12. Harvell, J.D., Kohler, S., Zhu, S. & Hernandez-boussard, T. (2004) High-Resolution Array-Based Comparative Genomic and Melanomas. Diagnostic Molecular Pathology 94305, 22-25.
13. Seykora, J.T., Jih, D., Elenitsas, R., Horng, W.-H. & Elder, D.E. (2003) Gene expression profiling of melanocytic lesions. The American Journal of dermatopathology 25, 6-11.
14. Talantov, D. et al. (2005) Novel genes associated with malignant melanoma but not benign melanocytic lesions. Clinical cancer research: an official journal of the American Association for Cancer Research 11, 7234-42.
15. Koh, S.S. et al. (2009) Molecular classification of melanomas and nevi using gene expression microarray signatures and formalin-fixed and paraffin-embedded tissue. Modern pathology: an official journal of the United States and Canadian Academy of Pathology, Inc. 22, 538-46.
16. Haqq, C. et al. (2005) The gene expression signatures of melanoma progression. Proc Natl Acad Sci U S A 102, 6092-6097.
17. Smith, A.P., Hoek, K. & Becker, D. (2005) Whole-genome expression profiling of the melanoma progression pathway reveals marked molecular differences between nevi/melanoma in situ and advanced-stage melanomas. Cancer biology & therapy 4, 1018-29.
18. Scatolini, M. et al. (2010) Altered molecular pathways in melanocytic lesions. International journal of cancer. Journal international du cancer 126, 1869-81.
19. Gerami, P. et al. (2009) Fluorescence in situ hybridization (FISH) as an ancillary diagnostic tool in the diagnosis of melanoma. The American journal of surgical pathology 33, 1146-56.
20. Vergier, B. et al. (2011) Fluorescence in situ hybridization, a diagnostic aid in ambiguous melanocytic tumors: European study of 113 cases. Mod Pathol. 24(5):613-23.
21. Gaiser, T. et al. (2010) Classifying ambiguous melanocytic lesions with FISH and correlation with clinical long-term follow up. Modern pathology: an official journal of the United States and Canadian Academy of Pathology, Inc 23, 413-9.
22. Alexandrescu, D.T. et al. (2010) Melanoma-specific marker expression in skin biopsy tissues as a tool to facilitate melanoma diagnosis. J Invest Dermatol 130, 1887-1892.
23. Kashani-Sabet, M. et al. ((2009) A multi-marker assay to distinguish malignant melanomas from benign nevi. Proc Natl Acad Sci U S A 106, 6268-6272.
24. Suzuki, I. & Motokawa, T. (2004) In situ hybridization: an informative technique for pigment cell researchers. Pigment cell research / sponsored by the European Society for Pigment Cell Research and the International Pigment Cell Society 17, 10-4.
25. Hanahan, D. & Weinberg, R.A. (2011) Hallmarks of Cancer: The Next Generation. Cell 144, 646-674.
26. Paik, S. et al. (2004) A multigene assay to predict recurrence of tamoxifen-treated, node-negative breast cancer. The New England journal of medicine 351, 2817-26.
27. van't Veer, L.J. et al. (2002) Gene expression profiling predicts clinical outcome of breast cancer. Nature 415, 530-6.
28. Finak, G. et al. (2008) Stromal gene expression predicts clinical outcome in breast cancer. Nature medicine 14, 518-27.
29. Hsu, M.-Y., Meier, F. & Herlyn, M. (2002) Melanoma development and progression: a conspiracy between tumor and host. Differentiation 70(9-10):522-36.
30. Masand, R.P. et al. (2011) Adenosquamous Carcinoma of the Head and Neck: Relationship to Human Papillomavirus and Review of the Literature. Head Neck Pathol. 5(2):108-16.
31. Ukpo, O.C. et al. High-Risk (2011) Human Papillomavirus E6/E7 mRNA Detection by a Novel In-Situ Hybridization Assay Strongly Correlates with p16 Expression and Patient Outcomes in Oropharyngeal Squamous Cell Carcinoma. USCAP Abstract 1207.
32. Smyth, G.K. Limma (2005) linear models for microarray data. Bioinformatics and Computational Biology Solutions using R and Bioconductor V. Carey, S. Dudoit, R. Irizarry, W. Huber (eds.), Springer, New York, pages 397-420.
33. Brown, L.F. et al. (1994) Osteopontin expression and distribution in human carcinomas. The American journal of pathology 145, 610-23.
34. Zhou, Y. et al. (2005) Osteopontin expression correlates with melanoma invasion. The Journal of Investigative Dermatology 124:1044-52.
35. Wai, P.Y. & Kuo, P.C. (2008) Osteopontin: regulation in tumor metastasis. Cancer metastasis reviews 27, 103-18.
36. Shevde, L.A., Das, S., Clark, D.W. & Samant, R.S. (2010) Osteopontin: an effector and an effect of tumor metastasis. Current molecular medicine 10:71-81.
37. El-Tanani, M.K. et al. (2006) The regulation and role of osteopontin in malignant transformation and cancer. Cytokine & growth factor reviews 17:463-74.
38. Sagara, J., Arata, T. & Taniguchi, S. (2009) Scapinin, the protein phosphatase 1 binding protein, enhances cell spreading and motility by interacting with the actin cytoskeleton. PLoS One 4, e4247.
39. Böni, R. et al. (1997) Ca(2+)-binding proteins S100A6 and S100B in primary cutaneous melanoma. Journal of cutaneous pathology 24, 76-80.
40. Mauerer, A. et al. (2011) Identification of new genes associated with melanoma. Experimental dermatology. 20(6):502-7
41. Soikkeli, J. et al. (2007) Systematic search for the best gene expression markers for melanoma micrometastasis detection. The Journal of pathology 213, 180-9.
42. Westekemper, H. et al. (2010) Expression of MCSP and PRAME in conjunctival melanoma. British Journal of Ophthalmology 94, 1322-1327.
43. Riker, A.I. et al. (2008) The gene expression profiles of primary and metastatic melanoma yields a transition point of tumor progression and metastasis. BMC medical genomics 1, 13.
44. Amatschek, S. et al. (2011) CXCL9 induces chemotaxis, chemorepulsion and endothelial barrier disruption through CXCR3-mediated activation of melanoma cells. British Journal of Cancer 104, 469-79.
45. Khaitan, D. et al. (2011) The Melanoma-Upregulated Long Noncoding RNA SPRY4-IT1 Modulates Apoptosis and Invasion. Cancer research 71(11):3852-62.
46. Balch, C.M. et al. (2004) An Evidence-based Staging System for Cutaneous Melanoma. CA: A Cancer Journal for Clinicians 54, 131-149.
47. Mocellin, S., Hoon, D.S.B., Pilati, P., Rossi, C.R. & Nitti, D. (2007) Sentinel lymph node molecular ultrastaging in patients with melanoma: a systematic review and meta-analysis of prognosis. Journal of clinical oncology 25, 1588-95.
48. Taback, B. et al. (2001) The clinical utility of multimarker RT-PCR in the detection of occult metastasis in patients with melanoma. Recent Results in Cancer Research. 158:78-92.

### EXAMPLES

### Example 1. Selection of PHACTR1, SPP1, and MLANA as Biomarkers for Detecting Melanoma

To identify the molecular events associated with melanoma progression and potential novel markers for early melanoma diagnosis, genome-wide gene expression microarrays were performed by several groups, and several datasets were made available through the Gene Expression Omnibus (GEO) database (http://(www.)ncbi.nlm.nih.gov/geo/). Although bioinformatic and statistical analysis had already been performed on the these datasets by the original authors, it was reasoned that combining results from multiple datasets, especially those from different microarray platforms, may lead to consistent, cross-validated candidate markers. Consequently, two different datasets comparing normal skin or benign nevi and malignant melanoma, GSE3189 (Affymetrix® GeneChip® HG-U133A, 22,000 genes) and GSE12391 (Agilent® Human Whole Genome Oligo Microaary, 41,000 genes) were selected, for candidate identification. GSE3189 contains data for 45 primary melanoma, 18 benign nevi and 7 normal skin specimens. GSE12391 contains a data series for 18 common nevi (CMN), 11 dysplastic nevi (DN), 8 radial (RGP) and 15 vertical (VGP) growth phase melanomas and 5 melanoma metastasis (MTS). These lesions represented different stages of melanoma progression according to a current model, which posits that melanoma initiates from dysplastic nevi, which then progresses to melanoma *in situ* or minimally invasive melanoma (RGP) to invasive melanoma (VGP) to metastatic melanoma (MTS) in a stepwise fashion.

The Limma linear models for microarrays were used to find genes differentially expressed between normal skin/benign nevi and melanomas. This algorithm uses empirical Bayes shrinkage of gene-wise residuals to ensure stable linear models when the number of samples is small, as is the case in these studies. PHACTR1 was the most and the second most strongly up-regulated gene in melanoma in GSE12391 and GSE3189, respectively (**Table 1**). The adjusted p values were after controlling for multiple hypothesis testing. The large difference in fold changes in these two datasets is likely due to the use of different microarray platforms and different data normalization schemes. Importantly, PHACTR1 demonstrated up-regulation in the early stage of RGP, whereas its expression in dysplastic nevi was similar to that in common nevi (**Fig. 4**), suggesting its potential utility to differentiate between dysplastic nevi and melanomas. Interestingly, PHACTR1 was not selected for validation in either of the two original studies, probably due to its lower ranking by the different statistical tests used.

**Table 1. Consistent up-regulation of PHACTR1 in melanoma in two microarray datasets.**

| **Gene** | **Dataset** | **Rank** | **log2(Fold Change)** | **t-statistic** | **Adjusted *P*** |
|---|---|---|---|---|---|
| PHACTR1 | GSE12391 | 1 | 1.62 | 7.91 | 3.52E-06 |
| PHACTR1 | GSE3189 | 2 | 5.32 | 19.15 | 7.24E-26 |

Therefore, PHACTR1 was selected as the top candidate. In addition, SPP1 and four other genes were chosen for validation on the basis of their differential gene expression in these datasets and literature support. Finally, two widely used melanocyte-specific markers, MLANA and TYR were evaluated. The which MLANA and TYR genes are expressed both in normal melanocytes and melanoma cells, but not in other cell types in the skin.

### Example 2. Validation of PHACTR1, SPP1, and MLANA as Biomarkers for Detecting Melanoma

RNAscope® assays using gene-specific probes against these eight selected candidate genes were performed on a series of FFPE tissue microarrays (TMAs) obtained from commercial vendors. The bacterial gene DapB was used as negative control. These TMAs contained normal skin, nevi and melanomas of different stages. TMAs are efficient means to test many candidate genes in multiple samples.

In these TMA experiments, MLANA had higher and more consistent expression in benign nevi and melanomas than TYR. Thus, MLANA was chosen as the marker for melanocytes and melanoma cells. It also served as a positive control for RNA integrity, since these TMAs were constructed from archival tissues from various times and sources and had variable quality in RNA preservation. Together, 70% of the melanocytic nevi and melanomas on the TMAs had MLANA-staining intensity scores above 2+ on a semi-quantitative scale and were considered as having acceptable quality, resulting in 78 melanomas and 19 nevi in the final sample set.

Among the 6 candidate genes differentially expressed between nevi and melanomas, two genes, PHACTR1 and SPP1, emerged as the strongest markers for melanoma and were detected at the early phase of melanoma (RGP). As shown in **Table 2**, 83% and 91% of the melanomas were positive for PHACTR1 and SPP1, respectively, whereas none of the 19 nevi had detectable signals for either marker. In combination, 95% of the melanomas were positive for at least one marker: 84% were positive for both, 12% and 4% were positive for either SPP1 or PHACTR1 alone, respectively.

**Table 2. Summary of three TMA studies combined.**

| **Sample Type** | **N** | **PHACTR1** | | **SPP1** | | **PHACTR1 or SPP1** | |
|---|---|---|---|---|---|---|---|
| | | **Positive** | **%** | **Positive** | **%** | **Positive** | **%** |
| **Melanoma** | 78 | 65 | 83% | 71 | 91% | 74 | 95% |
| **Nevi** | 19 | 0 | 0% | 0 | 0% | 0 | 0% |

Examples of staining patterns of MLANA, PHACTR1 and SPP1 in a benign nevus and a melanoma are shown in **Fig. 6****.** The benign nevus was completely negative for PHACTR1 and SPP1, whereas the melanoma was positive for both.

Interestingly, it was noticed that in addition to the strong positive staining of SPP1 in melanoma cells as shown in **Fig. 7****,** in certain cases of melanoma, SPP1 was detected primarily in individual cells scattered within the tumor region **(****Fig. 7****).** In a previous study using radioactive *in situ* hybridization, SPP1 mRNA was detected specifically in tumor-associated macrophages (TAMs) in multiple tumor types including melanoma, especially at the interface between tumor and stroma. Therefore, it was demonstrated two patterns of SPP1 expression: either predominantly in TAMs or in both TAMs and tumor cells. SPP1 protein expression in melanoma cells was also observed using immunohistochemistry.

### Example 3. Selection of Additional Biomarkers for Detecting Melanoma

Further improvements in RNAscope® Mela performance are highly desirable. First, MLANA was used as a positive control to help identify melanocytes (benign or malignant) and to assess quality of specimens. However, desmoplastic melanoma, representing 2-3% of all melanomas, is known to be negative for MLANA expression. A small fraction of other melanomas may also be negative for MLANA. Thus a negative MLANA staining result may be difficult to interpret. Second, PHACTR1 and SPP1 were negative in 5% of the melanomas, resulting in false negative classifications.

Therefore, it was sought to add additional markers to the 3-gene panel. First, two S100 genes, S100A6 and S100B, were added to MLANA to serve as positive control for assay procedure and sample quality control. S 100A6 and S100B are known to be expressed in desmoplastic melanomas which are usually nelan-A-negative. An example of RNAscope® detection of S100 in desmoplastic melanoma is shown in **Fig. 8****,** where MLANA staining was weakly positive, but the pooled probes for S100A6 and S100B detected strong signals. In a TMA study, S100A6 and S100B staining patterns were highly correlated but some cases showed strong signals only in one of them. Therefore, the probes for MLANA, S100A6 and S100B were pooled together to serve as positive controls for melanocytes.

Next, 9 additional candidate genes (PRAME, SILV, CXCL9, CXCL10, SPRY4-IT1, GDF15, KRT15, PTN, and CFH) that are differentially expressed between melanoma and benign nevus based on literature review were selected. RNAscope® assays for the newly selected genes were developed and the 9 genes were tested for their diagnostic performance in a TMA of 100 samples (77 melanoma and 23 nevi). All staining were scored on a 0-4 semi-quantitative scale. Using a score cutoff of 3 or higher (3+), only 43% of the samples met this criterion using MLANA alone, but 70% of the samples met this criterion using the MLANA/S100A6/S100B probe pool, confirming the added sensitivity of S100 genes in the positive control. Among the 9 new and 2 existing genes (PHACTR1 and SPP1), PHACTR1 and SPP1 remain the best two single markers, followed by PRAME and GDF15 (see **Fig. 9** for examples of RNAscope® staining), all showing positive staining in melanomas and negative staining in nevi, with no false positives **(Table 3**). The two chemokines CXCL9 and CXCL10 also showed 100% specificity but their sensitivity was low **(Table 3**). However, these two genes are stromal markers and expressed in tumor-associated endothelial cells (see **Fig. 9** for an example of RNAscope® staining). Since CXCL10 showed somewhat higher signals and sensitivity than CXCL9, it was chosen for further evaluation.

### Example 4. Evaluation of PHACTR1, SPP1, MLANA, PRAME, GDF15, CXCL10, S100A6 and S100B as Biomarkers for Detecting Melanoma

Potential combinations of PHACTR1, SPP1, MLANA, PRAME, GDF15, CXCL10, S100A6 and S100B genes to increase sensitivity while maintaining specificity at 100% **(Table 3).** Since both CXCL10 and SPP1 are expressed in the tumor-associated stroma, although in different cell types, it was sought to combine these two markers. Although combining SPP1 with CXCL10 did not lead to further increase in sensitivity compared to SPP1 alone, the combined macrophage- and endothelial-staining cells makes the assay more robust. Compared to the original proposed panel consisting of PHACTR1 and SPP1, adding PRAME, GDF15 and CXCL10 resulted in an increase in sensitivity from 95% to 98%, while maintaining 100% specificity. Interestingly, logistic regression with model selection resulted in the minimal combination of PRAME and SPP1, which had the same level of performance as the 5-gene panel. The combination of 5 genes of PHACTR1, SPP1, PRAME, GDF15 and CXCL10 demonstrated the potential of a reduced model for melanoma detection.

**Table 3. Classification performance of individual markers and their combinations.**

| **Pos. control=3+, nevi=8, mm=62** | **Sensitivity** | **Specificity** | **PPV** | **NPV** |
|---|---|---|---|---|
| **SILV** | 0.87 | 0.25 | 0.90 | 0.20 |
| **CXCL9** | 0.21 | 1.00 | 1.00 | 0.14 |
| **SPRY4.IT1** | 0.30 | 1.00 | 1.00 | 0.16 |
| **CXCL10** | 0.36 | 1.00 | 1.00 | 0.17 |
| **SPP1** | 0.95 | 1.00 | 1.00 | 0.73 |
| **PHACTR1** | 0.79 | 1.00 | 1.00 | 0.38 |
| **PRAME** | 0.72 | 1.00 | 1.00 | 0.32 |
| **GDF15** | 0.64 | 1.00 | 1.00 | 0.27 |
| **KRT15** | 0.95 | 0.63 | 0.95 | 0.63 |
| **CFH** | 0.39 | 1.00 | 1.00 | 0.18 |
| **PTN** | 0.97 | 0.75 | 0.97 | 0.75 |
| **PHACTR1.PRAME** | 0.89 | 1.00 | 1.00 | 0.53 |
| **PHACTR1.GDF15** | 0.89 | 1.00 | 1.00 | 0.53 |
| **PHACTR1.GDF15.PRAME** | 0.93 | 1.00 | 1.00 | 0.67 |
| **SPP1.CXCL10** | 0.95 | 1.00 | 1.00 | 0.73 |
| **PHACTR1.GDF15.PRAME.SPP1.CXCL10** | 0.98 | 1.00 | 1.00 | 0.89 |
| **PHACTR1.SPP1** | 0.95 | 1.00 | 1.00 | 0.73 |
| **PRAME.SPP1** | 0.98 | 1.00 | 1.00 | 0.89 |

We also performed an analysis between the expression levels of 5 genes of PHACTR1, SPP1, PRAME, GDF15 and CXCL10 and their relations to the diagnosis of melanoma. We tested for associations between the copy number of mRNA of each above gene and the presence of melanoma. We found that in patients with melanoma, the copy number of mRNA of PHACTR1, PRAME, GDF15 and CXCL10 genes could range from 1 to over 50. The copy number of mRNA of SPP1 gene can be even higher, ranging from 1 to over 100. We determined that 1 copy of mRNA can serve as a reliable threshold for determining the presence of melanoma in a tissue sample, meaning that if there is a single copy of mRNA of any of the 5 genes of PHACTR1, SPP1, PRAME, GDF15 and CXCL10, melanoma can be reliably predicted. We also found that if the threshold is raised to 3 copies of mRNA of any one of the 5 genes, the presence of melanoma can be predicted in certain.

## Claims

1. A method for diagnosing melanoma in a human subject suspected of melanoma, comprising determining the level of expression of integrin-binding phosphoprotein (SPP1) gene, or fragments thereof and preferentially expressed antigen in melanoma (PRAME) gene, or fragments thereof in a tissue sample from said human subject by *in situ* hybridization, wherein said tissue sample comprises a plurality of melanocytes, thereby diagnosing the presence of melanoma based on the expression levels in said tissue sample.

2. A method for providing a prognosis to a human subject who is at risk of developing melanoma recurrence, comprising determining the level of expression of integrin-binding phosphoprotein (SPP1) gene, or fragments thereof and preferentially expressed antigen in melanoma (PRAME) gene, or fragments thereof in a tissue sample from said human subject by *in situ* hybridization, wherein said tissue sample comprises a plurality of melanocytes, thereby providing prognosis based on the expression levels in said tissue sample.

3. The method of any of claim 1 or 2, wherein said determining the level of expression further comprises determining the level of expression of phosphatase and actin regulator 1 (PHACTR1) gene, or fragments thereof; growth differentiation factor 15 (GDF15) gene, or fragments thereof; and chemokine C-X-C motif ligand 10 (CXCL10) gene, or fragments thereof.

4. The method of any one of claims 1 to 3, wherein said tissue sample is skin.

5. The method of any one of claims 1 to 4, wherein said diagnosing, or said providing prognosis is based on a pattern of expression levels of said genes.

6. The method of claim 5, wherein said expression levels of said genes are determined individually; or are pooled and determined altogether.

7. The method of any one of claims 1 to 6, wherein said determining the level of expression comprises detecting the expression level of mRNA expressed from said genes; or detecting the expression level of polypeptides encoded by said genes.

8. The method of claim 7, wherein the upregulation of the mRNA expression of said genes, or the upregulation of the polypeptide expression of said genes is indicative of melanoma, or of recurrence of melanoma, or of risk of recurrence of melanoma.

9. A kit for the diagnosis or prognosis of melanoma, comprising target probes for the *in situ* hybridization detection of the level of expression of the integrin-binding phosphoprotein (SPP1) target gene, or fragments thereof, and preferentially expressed antigen in melanoma (PRAME) gene, or fragments thereof, wherein the target probes are polynucleotides that are capable of hybridizing to an RNA expressed from the target genes and capturing label probes to said RNA.

10. The kit of claim 9, wherein the kit further comprises target probes and label probes for the *in situ* hybridization detection of the expression level of the phosphatase and actin regulator 1 (PHACTR1) gene, or fragments thereof, growth differentiation factor 15 (GDF15) gene, or fragments thereof, and chemokine C-X-C motif ligand 10 (CXCL10) gene, or fragments thereof.

11. The kit of claim 9 or 10, further comprising preamplifiers or amplifiers, wherein an amplifier is a polynucleotide that is capable of hybridizing to multiple label probes and at least one target probe or a nucleic acid bound to a target probe, and wherein a preamplifier is a polynucleotide that serves as an intermediate between one or more target probes and amplifiers.

12. A kit for the diagnosis or prognosis of melanoma, comprising ligands or antibodies for detection of the level of target polypeptides, wherein said target polypeptides comprise a target polypeptide encoded by integrin-binding phosphoprotein (SPP1) gene, or fragments thereof; and a target polypeptide encoded by preferentially expressed antigen in melanoma (PRAME) gene, or fragments thereof.

13. The kit of claim 12, wherein said target polypeptides further comprise a target polypeptide encoded by phosphatase and actin regulator 1 (PHACTR1) gene, or fragments thereof; a target polypeptide encoded by growth differentiation factor 15 (GDF15) gene, or fragments thereof; and a target polypeptide encoded by chemokine C-X-C motif ligand 10 (CXCL10) gene, or fragments thereof.

14. A kit for the diagnosis or prognosis of melanoma, comprising oligonucleotides for the *in situ* hybridization detection of the expression level of the integrin-binding phosphoprotein (SPP1) target gene, or fragments thereof and preferentially expressed antigen in melanoma (PRAME) gene, or fragments thereof, wherein the oligonucleotides are capable of hybridizing to an RNA expressed from said target genes.

15. The kit of claim 14, wherein the kit further comprises oligonucleotides for the *in situ* hybridization detection of the expression level of the phosphatase and actin regulator 1 (PHACTR1) gene, or fragments thereof, growth differentiation factor 15 (GDF15) gene, or fragments thereof, and chemokine C-X-C motif ligand 10 (CXCL10) gene, or fragments thereof.

## Patentansprüche

1. Verfahren zum Diagnostizieren eines Melanoms in einem Menschen mit Verdacht auf ein Melanom, umfassend das Bestimmen des Expressionsniveaus von Integrin-bindendem Phosphoprotein (SPP1)-Gen oder Fragmenten davon und vorzugsweise exprimiertem Antigen in Melanom (PRAME)-Gen oder Fragmenten davon in einer Gewebeprobe des Menschen durch *in situ-*Hybridisierung, wobei die Gewebeprobe eine Vielzahl von Melanozyten umfasst, wodurch das Vorliegen von Melanom, basierend auf den Expressionsniveaus in der Gewebeprobe, diagnostiziert wird.

2. Verfahren zum Bereitstellen einer Prognose für einen Menschen, bei dem ein Risiko zum Entwickeln von Melanom-Wiederauftreten besteht, umfassend das Bestimmen des Expressionsniveaus von Integrin-bindendem Phosphoprotein (SPP1)-Gen oder Fragmenten davon und vorzugsweise exprimiertem Antigen im Melanom (PRAME)-Gen oder Fragmenten davon in einer Gewebeprobe des Menschen durch *in situ*-Hybridisierung, wobei die Gewebeprobe eine Vielzahl von Melanozyten umfasst, wodurch eine Prognose, basierend auf den Expressionsniveaus in der Gewebeprobe, bereitgestellt wird.

3. Verfahren nach einem von Anspruch 1 oder 2, wobei das Bestimmen des Expressionsniveaus ferner das Bestimmen des Expressionsniveaus von Phosphatase und Actin-Regulator 1 (PHACTR1)-Gen oder Fragmenten davon; von Wachstumsdifferenzierungsfaktor 15 (GDF15)-Gens oder Fragmente davon; und von Chemokin C-X-C Motivligand 10 (CXCL10)-Gens oder Fragmente davon umfasst.

4. Verfahren nach einem der Ansprüche 1 bis 3, wobei die Gewebeprobe Haut ist.

5. Verfahren nach einem der Ansprüche 1 bis 4, wobei das Diagnostizieren oder das Bereitstellen der Prognose auf einem Muster der Expressionsniveaus der Gene basiert.

6. Verfahren nach Anspruch 5, wobei die Expressionsniveaus der Gene einzeln bestimmt werden oder vereinigt und zusammen bestimmt werden.

7. Verfahren nach einem der Ansprüche 1 bis 6, wobei das Bestimmen des Expressionsniveaus das Nachweisen des Expressionsniveaus der von den Genen exprimierten mRNA oder das Nachweisen des Expressionsniveaus der durch die Gene kodierten Polypeptiden umfasst.

8. Verfahren nach Anspruch 7, wobei die Hochregulierung der mRNA-Expression der Gene oder die Hochregulierung der Polypeptidexpression der Gene für ein Melanom oder für das Wiederauftreten eines Melanoms oder für das Risiko des Wiederauftretens eines Melanoms hinweisend ist.

9. Kit zur Diagnose oder Prognose des Melanoms, umfassend Targetsonden für den *in situ*-Hybridisierungsnachweis des Expressionsniveaus des Integrin-bindenden Phosphoprotein (SPP1)-Targetgens oder Fragmente davon und des vorzugsweise exprimierten Antigens in Melanom (PRAME)-Targetgens oder Fragmente davon, wobei die Targetsonden Polynukleotide sind, die in der Lage sind, mit einer von den Targetgenen exprimierten RNA zu hybridisieren und Markierungssonden an der RNA einzufangen.

10. Kit nach Anspruch 9, wobei das Kit ferner Targetsonden und Markierungssonden für den *in situ*-Hybridisierungsnachweis des Expressionsgrades des Phosphatase und Actin-Regulator 1 (PHACTR1)-Gens oder Fragmenten davon, des Wachstumsdifferenzierungsfaktor 15 (GDF15)-Gens oder Fragmenten davon und des Chemokin C-X-C Motivligand 10 (CXCL10)-Gens oder Fragmenten davon umfasst.

11. Kit nach Anspruch 9 oder 10, ferner umfassend Preamplifier oder Amplifier, wobei ein Amplifier ein Polynukleotid ist, das in der Lage ist, mit mehreren Markierungssonden und mindestens einer Targetsonde oder einer an eine Targetsonde gebundenen Nukleinsäure zu hybridisieren, und wobei ein Preamplifier ein Polynukleotid ist, das als ein Zwischenprodukt zwischen einer oder mehreren Targetsonden und Amplifiern dient.

12. Kit zur Diagnose oder Prognose des Melanoms, umfassend Liganden oder Antikörper zum Nachweis der Menge an Target-Polypeptiden, wobei die Target-Polypeptide ein durch Integrin-bindendes Phosphoprotein (SPP1)-Gen oder Fragmente davon kodiertes Target-Polypeptid und ein durch vorzugsweise exprimiertes Antigen im Melanom (PRAME)-Gen oder Fragmente davon kodiertes Target-Polypeptid umfassen.

13. Kit nach Anspruch 12, wobei die Target-Polypeptide ferner ein durch das Phosphatase und Actin-Regulator 1 (PHACTR1)-Gen oder Fragmente davon kodiertes Target-Polypeptid; ein durch das Wachstumsdifferenzierungsfaktor 15 (GDF15)-Gen oder Fragmente davon kodiertes Target-Polypeptid; und ein durch das Chemokin C-X-C Motivligand 10 (CXCL10)-Gen oder Fragmente davon kodiertes Target-Polypeptid umfassen.

14. Kit zur Diagnose oder Prognose des Melanoms, umfassend Oligonukleotide für den *in situ*-Hybridisierungsnachweis des Expressionsniveaus von dem Integrin-bindenden Phosphoprotein (SPP1)-Targetgen oder Fragmente davon und das vorzugsweise exprimierte Antigen im Melanom (PRAME)-Targetgen oder Fragmente davon, wobei die Oligonukleotide in der Lage sind, mit einer von den Targetgenen exprimierten RNA zu hybridisieren.

15. Kit nach Anspruch 14, wobei das Kit ferner Oligonukleotide für den *in situ-*Hybridisierungsnachweis des Expressionsgrades von dem Phosphatase und Actin-Regulator 1 (PHACTR1)-Gen oder Fragmenten davon, Wachstumsdifferenzierungsfaktor 15 (GDF15)-Gen oder Fragmenten davon und Chemokin C-X-C Motivligand 10 (CXCL10)-Gen oder Fragmenten davon umfasst.

## Revendications

1. Procédé de diagnostic d'un mélanome chez un sujet humain suspecté d'avoir un mélanome, comprenant la détermination du niveau d'expression du gène de la phosphoprotéine se liant à l'intégrine (SPP1), ou de fragments de celui-ci et du gène de l'antigène préférentiellement exprimé dans un mélanome (PRAME), ou de fragments de celui-ci dans un échantillon de tissu provenant dudit sujet humain par hybridation *in situ,* dans lequel ledit échantillon de tissu comprend une pluralité de mélanocytes, pour ainsi diagnostiquer la présence d'un mélanome sur la base des niveaux d'expression dans ledit échantillon de tissu.

2. Procédé pour fournir un pronostic à un sujet humain qui est à risque de développer une récidive de mélanome, comprenant la détermination du niveau d'expression du gène de la phosphoprotéine se liant à l'intégrine (SPP1), ou de fragments de celui-ci et du gène de l'antigène préférentiellement exprimé dans un mélanome (PRAME), ou de fragments de celui-ci dans un échantillon de tissu provenant dudit sujet humain par hybridation *in situ,* dans lequel ledit échantillon de tissu comprend une pluralité de mélanocytes, pour ainsi fournir un pronostic sur la base des niveaux d'expression dans ledit échantillon de tissu.

3. Procédé selon une quelconque revendication 1 ou 2, dans lequel ladite détermination du niveau d'expression comprend en outre la détermination du niveau d'expression du gène du régulateur de phosphatase et d'actine 1 (PHACTR1), ou de fragments de celui-ci; du gène du facteur de différenciation de croissance 15 (GDF15), ou de fragments de celui-ci; et du gène du ligand du motif C-X-C de chimiokine 10 (CXCL10), ou de fragments de celui-ci.

4. Procédé selon l'une quelconque des revendications 1 à 3, dans lequel ledit échantillon de tissu est de la peau.

5. Procédé selon l'une quelconque des revendications 1 à 4, dans lequel ledit diagnostic, ou ladite fourniture d'un pronostic est basé sur un profil de niveaux d'expression desdits gènes.

6. Procédé selon la revendication 5, dans lequel lesdits niveaux d'expression desdits gènes sont déterminés individuellement; ou sont regroupés et déterminés entièrement.

7. Procédé selon l'une quelconque des revendications 1 à 6, dans lequel ladite détermination du niveau d'expression comprend la détection du niveau d'expression d'un ARNm exprimé à partir desdits gènes; ou la détection du niveau d'expression de polypeptides codés par lesdits gènes.

8. Procédé selon la revendication 7, dans lequel la régulation à la hausse de l'expression de l'ARNm desdits gènes, ou la régulation à la hausse de l'expression des polypeptides desdits gènes indique un mélanome, ou la récidive d'un mélanome, ou un risque de récidive de mélanome.

9. Kit pour le diagnostic ou le pronostic d'un mélanome, comprenant des sondes cibles pour la détection par hybridation *in situ* du niveau d'expression du gène cible de la phosphoprotéine se liant à l'intégrine (SPP1), ou de fragments de celui-ci et du gène cible de l'antigène préférentiellement exprimé dans un mélanome (PRAME), ou de fragments de celui-ci, dans lequel les sondes cibles sont des polynucléotides qui sont capables de s'hybrider à un ARN exprimé à partir des gènes cibles et de capturer des sondes de marquage sur ledit ARN.

10. Kit selon la revendication 9, dans lequel le kit comprend en outre des sondes cibles et des sondes de marquage pour la détection par hybridation *in situ* du niveau d'expression du gène du régulateur de phosphatase et d'actine 1 (PHACTR1), ou de fragments de celui-ci, du gène du facteur de différenciation de croissance 15 (GDF15), ou de fragments de celui-ci, et du gène du ligand du motif C-X-C de chimiokine 10 (CXCL10), ou de fragments de celui-ci.

11. Kit selon la revendication 9 ou 10, comprenant en outre des préamplificateurs ou des amplificateurs, dans lequel un amplificateur est un polynucléotide qui est capable de s'hybrider à de multiples sondes de marquage et à au moins une sonde cible ou à un acide nucléique lié à la sonde cible, et dans lequel un préamplificateur est un polynucléotide qui sert d'intermédiaire entre une ou plusieurs sondes cibles et des amplificateurs.

12. Kit pour le diagnostic ou le pronostic d'un mélanome, comprenant des ligands ou des anticorps pour la détection du taux de polypeptides cibles, dans lequel lesdits polypeptides cibles comprennent un polypeptide cible codé par le gène de la phosphoprotéine se liant à l'intégrine (SPP1), ou des fragments de celui-ci; et un polypeptide cible codé par le gène de l'antigène préférentiellement exprimé dans un mélanome (PRAME), ou des fragments de celui-ci.

13. Kit selon la revendication 12, dans lequel lesdits polypeptides cibles comprennent en outre un polypeptide cible codé par le gène du régulateur de phosphatase et d'actine 1 (PHACTR1), ou des fragments de celui-ci; un polypeptide cible codé par le gène du facteur de différenciation de croissance 15 (GDF15), ou des fragments de celui-ci; et un polypeptide cible codé par le gène du ligand du motif C-X-C de chimiokine 10 (CXCL10), ou des fragments de celui-ci.

14. Kit pour le diagnostic ou le pronostic d'un mélanome, comprenant des oligonucléotides pour la détection par hybridation *in situ* du niveau d'expression du gène cible de la phosphoprotéine se liant à l'intégrine (SPP1), ou de fragments de celui-ci et du gène cible de l'antigène préférentiellement exprimé dans un mélanome (PRAME), ou de fragments de celui-ci,dans lequel les oligonucléotides sont capables de s'hybrider à un ARN exprimé à partir desdits gènes cibles.

15. Kit selon la revendication 14, dans lequel le kit comprend en outre des oligonucléotides pour la détection par hybridation *in situ* du niveau d'expression du gène du régulateur de phosphatase et d'actine 1 (PHACTR1), ou de fragments de celui-ci, du gène du facteur de différenciation de croissance 15 (GDF15), ou de fragments de celui-ci, et du gène du ligand du motif C-X-C de chimiokine 10 (CXCL10), ou de fragments de celui-ci.


### SEQUENCE LISTING

<110> Advanced Cell Diagnostics
<120> BIOMARKERS FOR DIFFERENTIATING MELANOMA FROM
   BENIGN NEVUS IN THE SKIN
<130> 12790-019-228
<140>
   <141>
<150> 61/384,707
   <151> 2010-09-20
<150> 61/470,682
   <151> 2011-04-01
<160> 16
<170> FastSEQ for Windows Version 4.0
<210> 1
   <211> 2150
   <212> DNA
   <213> Homo sapiens
<220>
   <223> mRNA of phosphatase and actin regulator 1 (PHACTR1); Genbank accession No. NM_030948.1
<400> 1
   gtatgcatta ttcatcactg ttctaatcaa acaatcaaaa tcgtttactt tcaccgggaa 60
   tatttagcaa gatcaaagac actctggctg gaacggctct tgttttaccg ctgacagata 120
   gcctttgatt tttatttttt gcagtgttcc agtgttttct ctcaaaactc tgtgtttgga 180
   acatcaagga tggattatcc caaaatggat tattttctgg atgtagagtc tgctcacaga 240
   ctcttggatg ttgagtcagc tcaaagattc ttctacagtc aaggagctca agctcgccgg 300
   gcgaccctgc tcctgcctcc cacattaatg gcggcatcct cggaggatga tatagaccgg 360
   cggcccatcc ggagagtgcg ctccaagagc gacacgccgt acctcgcaga ggccaggatc 420
   tcctttaacc tgggggcagc tgaggaagtg gagaggctgg cggcgatgcg ttctgactcc 480
   ctcgtcccag gcacccacac cccacccatc cgcaggagaa gtaagtttgc caacctggga 540
   aggattttca agccttggaa atggaggaag aagaaaagcg aaaagttcaa acacacgtca 600
   gcagccctgg aaaggaaaat atctatgagg caaagcagag aagagctgat aaagcgagga 660
   gtcctgaagg aaatctatga taaagatggg gaactctcta tatccaatga agaggactcc 720
   ctagaaaatg ggcagtccct gagctccagc cagctgtctc tgcctgccct gtccgaaatg 780
   gagccagtcc caatgcccag ggatccctgc tcatatgagg tgctccaacc gtcagacatc 840
   atggatgggc cagatcctgg cgcccctgtg aaattgcctt gtctgccagt gaaactgtcg 900
   cctccgctac ctccaaagaa agtcatgatc tgtatgcccg tgggggggcc agacctctca 960
   ctggtgtcct acacagccca gaagagtggc cagcagggtg tggcccagca ccaccacact 1020
   gtcctgccct cccagatcca gcaccagctg cagtacggca gccacggcca gcacctcccc 1080
   tccaccaccg gctccctccc catgcacccc tcgggctgca gaatgataga cgagctcaac 1140
   aaaacgctgg ccatgaccat gcagaggctg gaaagctctg agcagcgggt cccctgttcc 1200
   acttcttacc acagctctgg gttgcactcg ggtgatgggg tcaccaaagc aggacctatg 1260
   ggccttccag aaataagaca agtgccaact gttgtgattg aatgtgatga caataaagaa 1320
   aatgtgcctc atgagtcaga ctacgaagac tcttcttgcc tgtatacaag agaagaggag 1380
   gaagaggagg aggacgaaga cgacgacagc tcattataca ccagctccct ggccatgaag 1440
   gtctgcagga aggactcctt agccatcaaa ctcagcaaca ggccctccaa gcgagagctg 1500
   gaagaaaaga acatccttcc caggcagacg gatgaggagc ggctggagct gaggcaacag 1560
   attggcacca agctcaccag gcggctgagc cagaggccaa ctgcagagga actggaacag 1620
   aggaacattt tgaaacctcg gaatgaacaa gaggaacagg aggagaagag agagatcaag 1680
   aggaggctaa cccgaaagct cagtcaaagg cccacggtgg aagagcttcg ggaaagaaag 1740
   atcctcatcc gcttcagtga ctacgtggag gtggctgacg ctcaggacta tgaccgcagg 1800
   gcagataagc cgtggacccg cctcaccgct gcagacaaag ctgccatccg aaaggagctc 1860
   aatgaattca aaagcactga gatggaagtt catgaattga gtagacactt aacaaggttt 1920
   caccgacctt aacagtcgaa ttcctcttga gtgctatgct gtcttcaaaa cataaattta 1980
   taagaaccat aagtgctggt atttattcac ttccccatta cgatgtaaat cttctgaact 2040
   gccttttttt taaaaagaag aaaaatcaag gaaacacaat caggatttta tgtgtgaaaa 2100
   cgcaaaagtg atggctcggc ggtccgagct gctggtccca cttctgacac            2150
<210> 2
   <211> 580
   <212> PRT
   <213> Homo sapiens
<220>
   <223> phosphatase and actin regulator 1 (PHACTR1); Genbank accession No. NP_001229577.1
<400> 2
<210> 3
   <211> 1220
   <212> DNA
   <213> Homo sapiens
<220>
   <223> mRNA of growth differentiation factor 15 (GDF15), Genbank accession No. NM_004864.2
<400> 3
   agtcccagct cagagccgca acctgcacag ccatgcccgg gcaagaactc aggacggtga 60
   atggctctca gatgctcctg gtgttgctgg tgctctcgtg gctgccgcat gggggcgccc 120
   tgtctctggc cgaggcgagc cgcgcaagtt tcccgggacc ctcagagttg cactccgaag 180
   actccagatt ccgagagttg cggaaacgct acgaggacct gctaaccagg ctgcgggcca 240
   accagagctg ggaagattcg aacaccgacc tcgtcccggc ccctgcagtc cggatactca 300
   cgccagaagt gcggctggga tccggcggcc acctgcacct gcgtatctct cgggccgccc 360
   ttcccgaggg gctccccgag gcctcccgcc ttcaccgggc tctgttccgg ctgtccccga 420
   cggcgtcaag gtcgtgggac gtgacacgac cgctgcggcg tcagctcagc cttgcaagac 480
   cccaggcgcc cgcgctgcac ctgcgactgt cgccgccgcc gtcgcagtcg gaccaactgc 540
   tggcagaatc ttcgtccgca cggccccagc tggagttgca cttgcggccg caagccgcca 600
   gggggcgccg cagagcgcgt gcgcgcaacg gggaccactg tccgctcggg cccgggcgtt 660
   gctgccgtct gcacacggtc cgcgcgtcgc tggaagacct gggctgggcc gattgggtgc 720
   tgtcgccacg ggaggtgcaa gtgaccatgt gcatcggcgc gtgcccgagc cagttccggg 780
   cggcaaacat gcacgcgcag atcaagacga gcctgcaccg cctgaagccc gacacggtgc 840
   cagcgccctg ctgcgtgccc gccagctaca atcccatggt gctcattcaa aagaccgaca 900
   ccggggtgtc gctccagacc tatgatgact tgttagccaa agactgccac tgcatatgag 960
   cagtcctggt ccttccactg tgcacctgcg cggaggacgc gacctcagtt gtcctgccct 1020
   gtggaatggg ctcaaggttc ctgagacacc cgattcctgc ccaaacagct gtatttatat 1080
   aagtctgtta tttattatta atttattggg gtgaccttct tggggactcg ggggctggtc 1140
   tgatggaact gtgtatttat ttaaaactct ggtgataaaa ataaagctgt ctgaactgtt 1200
   aaaaaaaaaa aaaaaaaaaa                                              1220
<210> 4
   <211> 308
   <212> PRT
   <213> Homo sapiens
<220>
   <223> growth differentiation factor 15 (GDF15), Genbank accession No. NP_004855.2
<400> 4
   Met Pro Gly Gln Glu Leu Arg Thr Val Asn Gly Ser Gln Met Leu Leu
    1               5                  10                  15
   Val Leu Leu Val Leu Ser Trp Leu Pro His Gly Gly Ala Leu Ser Leu
               20                  25                  30
   Ala Glu Ala Ser Arg Ala Ser Phe Pro Gly Pro Ser Glu Leu His Ser
           35                  40                  45
   Glu Asp Ser Arg Phe Arg Glu Leu Arg Lys Arg Tyr Glu Asp Leu Leu
       50                  55                  60
   Thr Arg Leu Arg Ala Asn Gln Ser Trp Glu Asp Ser Asn Thr Asp Leu
   65                  70                  75                  80
   Val Pro Ala Pro Ala Val Arg Ile Leu Thr Pro Glu Val Arg Leu Gly
                   85                  90                  95
   Ser Gly Gly His Leu His Leu Arg Ile Ser Arg Ala Ala Leu Pro Glu
               100                 105                 110
   Gly Leu Pro Glu Ala Ser Arg Leu His Arg Ala Leu Phe Arg Leu Ser
           115                 120                 125
   Pro Thr Ala Ser Arg Ser Trp Asp Val Thr Arg Pro Leu Arg Arg Gln
       130                 135                 140
   Leu Ser Leu Ala Arg Pro Gln Ala Pro Ala Leu His Leu Arg Leu Ser
   145                 150                 155                 160
   Pro Pro Pro Ser Gln Ser Asp Gln Leu Leu Ala Glu Ser Ser Ser Ala
                   165                 170                 175
   Arg Pro Gln Leu Glu Leu His Leu Arg Pro Gln Ala Ala Arg Gly Arg
               180                 185                 190
   Arg Arg Ala Arg Ala Arg Asn Gly Asp His Cys Pro Leu Gly Pro Gly
           195                 200                 205
   Arg Cys Cys Arg Leu His Thr Val Arg Ala Ser Leu Glu Asp Leu Gly
       210                 215                 220
   Trp Ala Asp Trp Val Leu Ser Pro Arg Glu Val Gln Val Thr Met Cys
   225                 230                 235                 240
   Ile Gly Ala Cys Pro Ser Gln Phe Arg Ala Ala Asn Met His Ala Gln
                   245                 250                 255
   Ile Lys Thr Ser Leu His Arg Leu Lys Pro Asp Thr Val Pro Ala Pro
               260                 265                 270
   Cys Cys Val Pro Ala Ser Tyr Asn Pro Met Val Leu Ile Gln Lys Thr
           275                 280                 285
   Asp Thr Gly Val Ser Leu Gln Thr Tyr Asp Asp Leu Leu Ala Lys Asp
       290                 295                 300
   Cys His Cys Ile
   305
<210> 5
   <211> 1524
   <212> DNA
   <213> Homo sapiens
<220>
   <223> mRNA of melan-A (MLANA); Genbank accession No. NM_005511.1
<400> 5
   agcagacaga ggactctcat taaggaaggt gtcctgtgcc ctgaccctac aagatgccaa 60
   gagaagatgc tcacttcatc tatggttacc ccaagaaggg gcacggccac tcttacacca 120
   cggctgaaga ggccgctggg atcggcatcc tgacagtgat cctgggagtc ttactgctca 180
   tcggctgttg gtattgtaga agacgaaatg gatacagagc cttgatggat aaaagtcttc 240
   atgttggcac tcaatgtgcc ttaacaagaa gatgcccaca agaagggttt gatcatcggg 300
   acagcaaagt gtctcttcaa gagaaaaact gtgaacctgt ggttcccaat gctccacctg 360
   cttatgagaa actctctgca gaacagtcac caccacctta ttcaccttaa gagccagcga 420
   gacacctgag acatgctgaa attatttctc tcacactttt gcttgaattt aatacagaca 480
   tctaatgttc tcctttggaa tggtgtagga aaaatgcaag ccatctctaa taataagtca 540
   gtgttaaaat tttagtaggt ccgctagcag tactaatcat gtgaggaaat gatgagaaat 600
   attaaattgg gaaaactcca tcaataaatg ttgcaatgca tgatactatc tgtgccagag 660
   gtaatgttag taaatccatg gtgttatttt ctgagagaca gaattcaagt gggtattctg 720
   gggccatcca atttctcttt acttgaaatt tggctaataa caaactagtc aggttttcga 780
   accttgaccg acatgaactg tacacagaat tgttccagta ctatggagtg ctcacaaagg 840
   atacttttac aggttaagac aaagggttga ctggcctatt tatctgatca agaacatgtc 900
   agcaatgtct ctttgtgctc taaaattcta ttatactaca ataatatatt gtaaagatcc 960
   tatagctctt tttttttgag atggagtttc gcttttgttg cccaggctgg agtgcaatgg 1020
   cgcgatcttg gctcaccata acctccgcct cccaggttca agcaattctc ctgccttagc 1080
   ctcctgagta gctgggatta caggcgtgcg ccactatgcc tgactaattt tgtagtttta 1140
   gtagagacgg ggtttctcca tgttggtcag gctggtctca aactcctgac ctcaggtgat 1200
   ctgcccgcct cagcctccca aagtgctgga attacaggcg tgagccacca cgcctggctg 1260
   gatcctatat cttaggtaag acatataacg cagtctaatt acatttcact tcaaggctca 1320
   atgctattct aactaatgac aagtattttc tactaaacca gaaattggta gaaggattta 1380
   aataagtaaa agctactatg tactgcctta gtgctgatgc ctgtgtactg ccttaaatgt 1440
   acctatggca atttagctct cttgggttcc caaatccctc tcacaagaat gtgcagaaga 1500
   aatcataaag gatcagagat tctg                                        1524
<210> 6
   <211> 118
   <212> PRT
   <213> Homo sapiens
<220>
   <223> melan-A (MLANA); Genbank accession No. NP_005502.1
<400> 6
   Met Pro Arg Glu Asp Ala His Phe Ile Tyr Gly Tyr Pro Lys Lys Gly
    1               5                  10                  15
   His Gly His Ser Tyr Thr Thr Ala Glu Glu Ala Ala Gly Ile Gly Ile
               20                  25                  30
   Leu Thr Val Ile Leu Gly Val Leu Leu Leu Ile Gly Cys Trp Tyr Cys
           35                  40                  45
   Arg Arg Arg Asn Gly Tyr Arg Ala Leu Met Asp Lys Ser Leu His Val
       50                  55                  60
   Gly Thr Gln Cys Ala Leu Thr Arg Arg Cys Pro Gln Glu Gly Phe Asp
   65                  70                  75                  80
   His Arg Asp Ser Lys Val Ser Leu Gln Glu Lys Asn Cys Glu Pro Val
                   85                  90                  95
   Val Pro Asn Ala Pro Pro Ala Tyr Glu Lys Leu Ser Ala Glu Gln Ser
               100                 105                 110
   Pro Pro Pro Tyr Ser Pro
           115
<210> 7
   <211> 1184
   <212> DNA
   <213> Homo sapiens
<220>
   <223> mRNA of chemokine (C-X-C motif) ligand 10 (CXCL10); Genbank accession No. NM_001565
<210> 8
   <211> 98
   <212> PRT
   <213> Homo sapiens
<220>
   <223> chemokine (C-X-C motif) ligand 10 (CXCL10); Genbank accession No. NP_001556.2
<400> 8
   Met Asn Gln Thr Ala Ile Leu Ile Cys Cys Leu Ile Phe Leu Thr Leu
    1               5                  10                  15
   Ser Gly Ile Gln Gly Val Pro Leu Ser Arg Thr Val Arg Cys Thr Cys
               20                  25                  30
   Ile Ser Ile Ser Asn Gln Pro Val Asn Pro Arg Ser Leu Glu Lys Leu
           35                  40                  45
   Glu Ile Ile Pro Ala Ser Gln Phe Cys Pro Arg Val Glu Ile Ile Ala
       50                  55                  60
   Thr Met Lys Lys Lys Gly Glu Lys Arg Cys Leu Asn Pro Glu Ser Lys
   65                  70                  75                  80
   Ala Ile Lys Asn Leu Leu Lys Ala Val Ser Lys Glu Arg Ser Lys Arg
                   85                  90                  95
   Ser Pro
<210> 9
   <211> 2162
   <212> DNA
   <213> Homo sapiens
<220>
   <223> mRNA of preferentially expressed antigen in melanoma (PRAME), Genbank accession No. NM_006115.3
<400> 9
   cgagttccgg cgaggcttca gggtacagct cccccgcagc cagaagccgg gcctgcagcg 60
   cctcagcacc gctccgggac accccacccg cttcccaggc gtgacctgtc aacagcaact 120
   tcgcggtgtg gtgaactctc tgaggaaaaa ccattttgat tattactctc agacgtgcgt 180
   ggcaacaagt gactgagacc tagaaatcca agcgttggag gtcctgaggc cagcctaagt 240
   cgcttcaaaa tggaacgaag gcgtttgtgg ggttccattc agagccgata catcagcatg 300
   agtgtgtgga caagcccacg gagacttgtg gagctggcag ggcagagcct gctgaaggat 360
   gaggccctgg ccattgccgc cctggagttg ctgcccaggg agctcttccc gccactcttc 420
   atggcagcct ttgacgggag acacagccag accctgaagg caatggtgca ggcctggccc 480
   ttcacctgcc tccctctggg agtgctgatg aagggacaac atcttcacct ggagaccttc 540
   aaagctgtgc ttgatggact tgatgtgctc cttgcccagg aggttcgccc caggaggtgg 600
   aaacttcaag tgctggattt acggaagaac tctcatcagg acttctggac tgtatggtct 660
   ggaaacaggg ccagtctgta ctcatttcca gagccagaag cagctcagcc catgacaaag 720
   aagcgaaaag tagatggttt gagcacagag gcagagcagc ccttcattcc agtagaggtg 780
   ctcgtagacc tgttcctcaa ggaaggtgcc tgtgatgaat tgttctccta cctcattgag 840
   aaagtgaagc gaaagaaaaa tgtactacgc ctgtgctgta agaagctgaa gatttttgca 900
   atgcccatgc aggatatcaa gatgatcctg aaaatggtgc agctggactc tattgaagat 960
   ttggaagtga cttgtacctg gaagctaccc accttggcga aattttctcc ttacctgggc 1020
   cagatgatta atctgcgtag actcctcctc tcccacatcc atgcatcttc ctacatttcc 1080
   ccggagaagg aagagcagta tatcgcccag ttcacctctc agttcctcag tctgcagtgc 1140
   ctgcaggctc tctatgtgga ctctttattt ttccttagag gccgcctgga tcagttgctc 1200
   aggcacgtga tgaacccctt ggaaaccctc tcaataacta actgccggct ttcggaaggg 1260
   gatgtgatgc atctgtccca gagtcccagc gtcagtcagc taagtgtcct gagtctaagt 1320
   ggggtcatgc tgaccgatgt aagtcccgag cccctccaag ctctgctgga gagagcctct 1380
   gccaccctcc aggacctggt ctttgatgag tgtgggatca cggatgatca gctccttgcc 1440
   ctcctgcctt ccctgagcca ctgctcccag cttacaacct taagcttcta cgggaattcc 1500
   atctccatat ctgccttgca gagtctcctg cagcacctca tcgggctgag caatctgacc 1560
   cacgtgctgt atcctgtccc cctggagagt tatgaggaca tccatggtac cctccacctg 1620
   gagaggcttg cctatctgca tgccaggctc agggagttgc tgtgtgagtt ggggcggccc 1680
   agcatggtct ggcttagtgc caacccctgt cctcactgtg gggacagaac cttctatgac 1740
   ccggagccca tcctgtgccc ctgtttcatg cctaactagc tgggtgcaca tatcaaatgc 1800
   ttcattctgc atacttggac actaaagcca ggatgtgcat gcatcttgaa gcaacaaagc 1860
   agccacagtt tcagacaaat gttcagtgtg agtgaggaaa acatgttcag tgaggaaaaa 1920
   acattcagac aaatgttcag tgaggaaaaa aaggggaagt tggggatagg cagatgttga 1980
   cttgaggagt taatgtgatc tttggggaga tacatcttat agagttagaa atagaatctg 2040
   aatttctaaa gggagattct ggcttgggaa gtacatgtag gagttaatcc ctgtgtagac 2100
   tgttgtaaag aaactgttga aaataaagag aagcaatgtg aagcaaaaaa aaaaaaaaaa 2160
   aa                                                                2162
<210> 10
   <211> 509
   <212> PRT
   <213> Homo sapiens
<220>
   <223> preferentially expressed antigen in melanoma (PRAME), Genbank accession No. NP_006106.1
<400> 10
   Met Glu Arg Arg Arg Leu Trp Gly Ser Ile Gln Ser Arg Tyr Ile Ser
    1               5                  10                  15
   Met Ser Val Trp Thr Ser Pro Arg Arg Leu Val Glu Leu Ala Gly Gln
               20                  25                  30
   Ser Leu Leu Lys Asp Glu Ala Leu Ala Ile Ala Ala Leu Glu Leu Leu
           35                  40                  45
   Pro Arg Glu Leu Phe Pro Pro Leu Phe Met Ala Ala Phe Asp Gly Arg
       50                  55                  60
   His Ser Gln Thr Leu Lys Ala Met Val Gln Ala Trp Pro Phe Thr Cys
   65                  70                  75                  80
   Leu Pro Leu Gly Val Leu Met Lys Gly Gln His Leu His Leu Glu Thr
                   85                  90                  95
   Phe Lys Ala Val Leu Asp Gly Leu Asp Val Leu Leu Ala Gln Glu Val
               100                 105                 110
   Arg Pro Arg Arg Trp Lys Leu Gln Val Leu Asp Leu Arg Lys Asn Ser
           115                 120                 125
   His Gln Asp Phe Trp Thr Val Trp Ser Gly Asn Arg Ala Ser Leu Tyr
       130                 135                 140
   Ser Phe Pro Glu Pro Glu Ala Ala Gln Pro Met Thr Lys Lys Arg Lys
   145                 150                 155                 160
   Val Asp Gly Leu Ser Thr Glu Ala Glu Gln Pro Phe Ile Pro Val Glu
                   165                 170                 175
   Val Leu Val Asp Leu Phe Leu Lys Glu Gly Ala Cys Asp Glu Leu Phe
               180                 185                 190
   Ser Tyr Leu Ile Glu Lys Val Lys Arg Lys Lys Asn Val Leu Arg Leu
           195                 200                 205
   Cys Cys Lys Lys Leu Lys Ile Phe Ala Met Pro Met Gln Asp Ile Lys
       210                 215                 220
   Met Ile Leu Lys Met Val Gln Leu Asp Ser Ile Glu Asp Leu Glu Val
   225                 230                 235                 240
   Thr Cys Thr Trp Lys Leu Pro Thr Leu Ala Lys Phe Ser Pro Tyr Leu
                   245                 250                 255
   Gly Gln Met Ile Asn Leu Arg Arg Leu Leu Leu Ser His Ile His Ala
               260                 265                 270
   Ser Ser Tyr Ile Ser Pro Glu Lys Glu Glu Gln Tyr Ile Ala Gln Phe
           275                 280                 285
   Thr Ser Gln Phe Leu Ser Leu Gln Cys Leu Gln Ala Leu Tyr Val Asp
       290                 295                 300
   Ser Leu Phe Phe Leu Arg Gly Arg Leu Asp Gln Leu Leu Arg His Val
   305                 310                 315                 320
   Met Asn Pro Leu Glu Thr Leu Ser Ile Thr Asn Cys Arg Leu Ser Glu
                   325                 330                 335
   Gly Asp Val Met His Leu Ser Gln Ser Pro Ser Val Ser Gln Leu Ser
               340                 345                 350
   Val Leu Ser Leu Ser Gly Val Met Leu Thr Asp Val Ser Pro Glu Pro
           355                 360                 365
   Leu Gln Ala Leu Leu Glu Arg Ala Ser Ala Thr Leu Gln Asp Leu Val
       370                 375                 380
   Phe Asp Glu Cys Gly Ile Thr Asp Asp Gln Leu Leu Ala Leu Leu Pro
   385                 390                 395                 400
   Ser Leu Ser His Cys Ser Gln Leu Thr Thr Leu Ser Phe Tyr Gly Asn
                   405                 410                 415
   Ser Ile Ser Ile Ser Ala Leu Gln Ser Leu Leu Gln His Leu Ile Gly
               420                 425                 430
   Leu Ser Asn Leu Thr His Val Leu Tyr Pro Val Pro Leu Glu Ser Tyr
           435                 440                 445
   Glu Asp Ile His Gly Thr Leu His Leu Glu Arg Leu Ala Tyr Leu His
       450                 455                 460
   Ala Arg Leu Arg Glu Leu Leu Cys Glu Leu Gly Arg Pro Ser Met Val
   465                 470                 475                 480
   Trp Leu Ser Ala Asn Pro Cys Pro His Cys Gly Asp Arg Thr Phe Tyr
                   485                 490                 495
   Asp Pro Glu Pro Ile Leu Cys Pro Cys Phe Met Pro Asn
               500                 505
<210> 11
   <211> 683
   <212> DNA
   <213> Homo sapiens
<220>
   <223> mRNA of S100 calcium binding protein A6 (S100A6); Genbank accession No. NM_014624.3
<400> 11
   ctgcgcagat gaggggagac tcgtcaccag gcgtgcagtg ggcactgctg ggctccccca 60
   tcccgtccta acccggaaca gccccgggca ggaggcgtgg aaagtcgagg gggtaaaccg 120
   cgaatgtgcg ttgtgtaagc cacggcgcag ggtggggcgc gggcgggact tgggcgggcg 180
   gggtgggctt ggccgagctg gcctccgggg caccgaccgc tataaggcca gtcggactgc 240
   gacacagccc atcccctcga ccgctcgcgt cgcatttggc cgcctcccta ccgctccaag 300
   cccagccctc agccatggca tgccccctgg atcaggccat tggcctcctc gtggccatct 360
   tccacaagta ctccggcagg gagggtgaca agcacaccct gagcaagaag gagctgaagg 420
   agctgatcca gaaggagctc accattggct cgaagctgca ggatgctgaa attgcaaggc 480
   tgatggaaga cttggaccgg aacaaggacc aggaggtgaa cttccaggag tatgtcacct 540
   tcctgggggc cttggctttg atctacaatg aagccctcaa gggctgaaaa taaataggga 600
   agatggagac accctctggg ggtcctctct gagtcaaatc cagtggtggg taattgtaca 660
   ataaattttt tttggtcaaa ttt                                         683
<210> 12
   <211> 90
   <212> PRT
   <213> Homo sapiens
<220>
   <223> S100 calcium binding protein A6 (S100A6); Genbank accession No. NP_055439.1
<400> 12
   Met Ala Cys Pro Leu Asp Gln Ala Ile Gly Leu Leu Val Ala Ile Phe
    1              5                   10                  15
   His Lys Tyr Ser Gly Arg Glu Gly Asp Lys His Thr Leu Ser Lys Lys
               20                  25                  30
   Glu Leu Lys Glu Leu Ile Gln Lys Glu Leu Thr Ile Gly Ser Lys Leu
           35                  40                  45
   Gln Asp Ala Glu Ile Ala Arg Leu Met Glu Asp Leu Asp Arg Asn Lys
       50                  55                  60
   Asp Gln Glu Val Asn Phe Gln Glu Tyr Val Thr Phe Leu Gly Ala Leu
   65                  70                  75                  80
   Ala Leu Ile Tyr Asn Glu Ala Leu Lys Gly
                   85                  90
<210> 13
   <211> 1135
   <212> DNA
   <213> Homo sapiens
<220>
   <223> mRNA of S100 calcium binding protein B (S100B); Genbank accession No. NM_006272.2
<400> 13
   gggcagaggg aataagaggc tgcctctgcc caccagtcct gccgcccagg acccgcagca 60
   gagacgacgc ctgcagcaag gagaccagga aggggtgaga caaggaagag gatgtctgag 120
   ctggagaagg ccatggtggc cctcatcgac gttttccacc aatattctgg aagggaggga 180
   gacaagcaca agctgaagaa atccgaactg aaggagctca tcaacaatga gctttcccat 240
   ttcttagagg aaatcaaaga gcaggaggtt gtggacaaag tcatggaaac actggacaat 300
   gatggagacg gcgaatgtga cttccaggaa ttcatggcct ttgttgccat ggttactact 360
   gcctgccacg agttctttga acatgagtga gattagaaag cagccaaacc tttcctgtaa 420
   cagagacggt catgcaagaa agcagacagc aagggcttgc agcctagtag gagctgagct 480
   ttccagccgt gttgtagcta attaggaagc ttgatttgct ttgtgattga aaaattgaaa 540
   acctctttcc aaaggctgtt ttaacggcct gcatcattct ttctgctata ttaggcctgt 600
   gtgtaagctg actggcccca gggactcttg ttaacagtaa cttaggagtc aggtctcagt 660
   gataaagcgt gcaccgtgca gcccgccatg gccgtgtaga ccctaacccg gagggaaccc 720
   tgactacaga aattaccccg gggcaccctt aaaacttcca ctacctttaa aaaacaaagc 780
   cttatccagc attatttgaa aacactgctg ttctttaaat gcgttcctca tccatgcaga 840
   taacagctgg ttggccggtg tggccctgca agggcgtggt ggcttcggcc tgcttcccgg 900
   gatgcgcctg atcaccaggt gaacgctcag cgctggcagc gctcctggaa aaagcaactc 960
   catcagaact cgcaatccga gccagctctg ggggctccag cgtggcctcc gtgacccatg 1020
   cgattcaagt cgcggctgca ggatccttgc ctccaacgtg cctccagcac atgcggcttc 1080
   cgagggcact accgggggct ctgagccacc gcgagggcct gcgttcaata aaaag      1135
<210> 14
   <211> 92
   <212> PRT
   <213> Homo sapiens
<220>
   <223> S100 calcium binding protein B (S100B); Genbank accession No. NP_006263.1
<400> 14
   Met Ser Glu Leu Glu Lys Ala Met Val Ala Leu Ile Asp Val Phe His
    1               5                  10                  15
   Gln Tyr Ser Gly Arg Glu Gly Asp Lys His Lys Leu Lys Lys Ser Glu
               20                  25                  30
   Leu Lys Glu Leu Ile Asn Asn Glu Leu Ser His Phe Leu Glu Glu Ile
           35                  40                  45
   Lys Glu Gln Glu Val Val Asp Lys Val Met Glu Thr Leu Asp Asn Asp
       50                  55                  60
   Gly Asp Gly Glu Cys Asp Phe Gln Glu Phe Met Ala Phe Val Ala Met
   65                  70                  75                  80
   Val Thr Thr Ala Cys His Glu Phe Phe Glu His Glu
                   85                  90
<210> 15
   <211> 1616
   <212> DNA
   <213> Homo sapiens
<220>
   <223> mRNA of secreted phosphoprotein 1 (SPP1), transcript variant 2; Genbank accession No. NM_000582.2
<400> 15
   ctccctgtgt tggtggagga tgtctgcagc agcatttaaa ttctgggagg gcttggttgt 60
   cagcagcagc aggaggaggc agagcacagc atcgtcggga ccagactcgt ctcaggccag 120
   ttgcagcctt ctcagccaaa cgccgaccaa ggaaaactca ctaccatgag aattgcagtg 180
   atttgctttt gcctcctagg catcacctgt gccataccag ttaaacaggc tgattctgga 240
   agttctgagg aaaagcagct ttacaacaaa tacccagatg ctgtggccac atggctaaac 300
   cctgacccat ctcagaagca gaatctccta gccccacaga cccttccaag taagtccaac 360
   gaaagccatg accacatgga tgatatggat gatgaagatg atgatgacca tgtggacagc 420
   caggactcca ttgactcgaa cgactctgat gatgtagatg acactgatga ttctcaccag 480
   tctgatgagt ctcaccattc tgatgaatct gatgaactgg tcactgattt tcccacggac 540
   ctgccagcaa ccgaagtttt cactccagtt gtccccacag tagacacata tgatggccga 600
   ggtgatagtg tggtttatgg actgaggtca aaatctaaga agtttcgcag acctgacatc 660
   cagtaccctg atgctacaga cgaggacatc acctcacaca tggaaagcga ggagttgaat 720
   ggtgcataca aggccatccc cgttgcccag gacctgaacg cgccttctga ttgggacagc 780
   cgtgggaagg acagttatga aacgagtcag ctggatgacc agagtgctga aacccacagc 840
   cacaagcagt ccagattata taagcggaaa gccaatgatg agagcaatga gcattccgat 900
   gtgattgata gtcaggaact ttccaaagtc agccgtgaat tccacagcca tgaatttcac 960
   agccatgaag atatgctggt tgtagacccc aaaagtaagg aagaagataa acacctgaaa 1020
   tttcgtattt ctcatgaatt agatagtgca tcttctgagg tcaattaaaa ggagaaaaaa 1080
   tacaatttct cactttgcat ttagtcaaaa gaaaaaatgc tttatagcaa aatgaaagag 1140
   aacatgaaat gcttctttct cagtttattg gttgaatgtg tatctatttg agtctggaaa 1200
   taactaatgt gtttgataat tagtttagtt tgtggcttca tggaaactcc ctgtaaacta 1260
   aaagcttcag ggttatgtct atgttcattc tatagaagaa atgcaaacta tcactgtatt 1320
   ttaatatttg ttattctctc atgaatagaa atttatgtag aagcaaacaa aatactttta 1380
   cccacttaaa aagagaatat aacattttat gtcactataa tcttttgttt tttaagttag 1440
   tgtatatttt gttgtgatta tctttttgtg gtgtgaataa atcttttatc ttgaatgtaa 1500
   taagaatttg gtggtgtcaa ttgcttattt gttttcccac ggttgtccag caattaataa 1560
   aacataacct tttttactgc ctaaaaaaaa aaaaaaaaaa aaaaaaaaaa aaaaaa     1616
<210> 16
   <211> 314
   <212> PRT
   <213> Homo sapiens
<220>
   <223> secreted phosphoprotein 1 (SPP1), transcript variant 2; Genbank accession No. NP_001035147.1
<400> 16
   Met Arg Ile Ala Val Ile Cys Phe Cys Leu Leu Gly Ile Thr Cys Ala
    1               5                  10                  15
   Ile Pro Val Lys Gln Ala Asp Ser Gly Ser Ser Glu Glu Lys Gln Leu
               20                  25                  30
   Tyr Asn Lys Tyr Pro Asp Ala Val Ala Thr Trp Leu Asn Pro Asp Pro
           35                  40                  45
   Ser Gln Lys Gln Asn Leu Leu Ala Pro Gln Asn Ala Val Ser Ser Glu
       50                  55                  60
   Glu Thr Asn Asp Phe Lys Gln Glu Thr Leu Pro Ser Lys Ser Asn Glu
   65                  70                  75                  80
   Ser His Asp His Met Asp Asp Met Asp Asp Glu Asp Asp Asp Asp His
                   85                  90                  95
   Val Asp Ser Gln Asp Ser Ile Asp Ser Asn Asp Ser Asp Asp Val Asp
               100                 105                 110
   Asp Thr Asp Asp Ser His Gln Ser Asp Glu Ser His His Ser Asp Glu
           115                 120                 125
   Ser Asp Glu Leu Val Thr Asp Phe Pro Thr Asp Leu Pro Ala Thr Glu
       130                 135                 140
   Val Phe Thr Pro Val Val Pro Thr Val Asp Thr Tyr Asp Gly Arg Gly
   145                 150                 155                 160
   Asp Ser Val Val Tyr Gly Leu Arg Ser Lys Ser Lys Lys Phe Arg Arg
                   165                 170                 175
   Pro Asp Ile Gln Tyr Pro Asp Ala Thr Asp Glu Asp Ile Thr Ser His
               180                 185                 190
   Met Glu Ser Glu Glu Leu Asn Gly Ala Tyr Lys Ala Ile Pro Val Ala
           195                 200                 205
   Gln Asp Leu Asn Ala Pro Ser Asp Trp Asp Ser Arg Gly Lys Asp Ser
       210                 215                 220
   Tyr Glu Thr Ser Gln Leu Asp Asp Gln Ser Ala Glu Thr His Ser His
   225                 230                 235                 240
   Lys Gln Ser Arg Leu Tyr Lys Arg Lys Ala Asn Asp Glu Ser Asn Glu
                   245                 250                 255
   His Ser Asp Val Ile Asp Ser Gln Glu Leu Ser Lys Val Ser Arg Glu
               260                 265                 270
   Phe His Ser His Glu Phe His Ser His Glu Asp Met Leu Val Val Asp
           275                 280                 285
   Pro Lys Ser Lys Glu Glu Asp Lys His Leu Lys Phe Arg Ile Ser His
       290                 295                 300
   Glu Leu Asp Ser Ala Ser Ser Glu Val Asn
   305                 310